# EUROPEAN PATENT APPLICATION

(11) **EP 3 613 411 A1**
(43) Date of publication of application: **26.02.2020**
(21) Application number: 18788547.0
(22) Date of filing: 18.04.2018
(51) Int. Cl.: A61K 8/81, A61K 8/19, A61K 8/25, A61K 8/26, A61K 8/29, A61K 8/31, A61K 8/34, A61K 8/35, A61K 8/73, A61K 8/87, A61K 8/891, A61Q 1/02, B05B 5/025, B05D 1/04

(54) **METHOD FOR PRODUCING COATING**

(30) Priority: 18.04.2017 JP 2017082073
(71) Applicant: Kao Corporation, Chuo-ku Tokyo 103-8210 (JP)
(72) Inventor: ITO, Motoaki, Odawara-shi Kanagawa 250-0002 (JP)
(74) Representative: Hoffmann Eitle
(86) International application number: PCT/JP2018/015978
(87) International publication number: WO 2018/194084

(57) **Abstract**

Provided is a method for preventing color unevenness, white float and makeup collapse of a powder-containing cosmetic such as a foundation, applied to the skin.

A method for producing a coating on skin, comprising a step of electrostatically spraying a composition comprising component (a), component (b) and component (c) directly onto the skin:
(a) one or more volatile substances selected from the group consisting of water, an alcohol and a ketone,
(b) a polymer having a film-forming ability, and
(c) a powder.

## Description

### [Field of the Invention]

The present invention relates to a method for producing a cosmetic coating on the skin.

### [Background of the invention]

Various methods are known for forming coatings by electrostatic spraying. For example, Patent Literature 1 discloses a method for treating skin comprising electrostatically spraying the skin with a composition. The composition used in the method contains a liquid- insulating material, a conductive material, a particulate powder material, and a thickener. Typically, a cosmetic product or skincare composition containing a pigment is used as the composition. Specifically, a cosmetic foundation is used as the composition. That is, the inventions described in Patent Literature 1 primarily envision cosmetic purposes by electrostatically spraying a cosmetic foundation to cosmetically decorate the skin.

Patent Literature 2 discloses disposable cartridges for use in electrostatic spraying devices for cosmetics. The electrostatic spraying device is of a hand-held and self-contained type. This electrostatic spraying device is used for spraying cosmetic foundations in the same manner as in the Patent Literature 1 above.
(Patent Literature 1) JP-A-2006-104211
(Patent Literature 2) JP-A-2003-507165

### [Summary of the Invention]

The present invention provides a method for producing a coating on skin, comprising a step of electrostatically spraying a composition comprising component (a), component (b) and component (c) directly onto the skin:
(a) one or more volatile substances selected from the group consisting of water, an alcohol and a ketone,
(b) a polymer having a film-forming ability, and
(c) a powder.

### [Brief Description of the Drawings]

FIG. 1 is a schematic diagram showing a configuration of an electrostatic spraying device suitable for use in the present invention.
FIG. 2 is a schematic diagram showing a state in which an electrostatic spraying method is performed by using an electrostatic spraying device.

### [Detailed Description of the Invention]

Powder-containing cosmetics, typified by foundation and sunscreen cosmetics, may cause discoloration or white float when applied to the skin, and may cause cosmetic discoloration to be noticeable over time. Electrostatic spraying according to the methods described in Patent Literatures 1 and 2 does not improve the color unevenness, whiteness and cosmetic fastness sufficiently.

Thus, the present invention provides a method for preventing the coloration, white float, fading, etc. of powder-containing cosmetics, such as foundations, applied to the skin.

As a result of extensive studies, the present inventor has found that when a composition containing a particular volatile substance, a polymer having a film-forming ability, and a powder is electrostatically sprayed on the skin, a porous coating bearing the powder is formed on the skin, and the coating is uniformly dispersed and adhered to the surface of the skin hills, skin grooves and skin pores, the generation of unevenness of color and whiteness is suppressed, and it is difficult to make the composition cosmetic over time, thereby completing the present invention.

The coating formed on the skin by the method of the present invention suppresses the powder from agglomerating on the skin immediately after application and resulting in cosmetic kinking. In addition, the coating adheres uniformly to the skin hills, skin grooves and skin pores, and does not cause uneven color or white float.

In the present invention, a composition which is electrostatically sprayed directly onto the skin (hereinafter may be referred to as a spraying composition) is a composition which comprises the following components (a), (b) and (c):
(a) one or more volatile substances selected from the group consisting of water, an alcohol and a ketone,
(b) a polymer having a film-forming ability, and
(c) a powder.

The (a) volatile substance represents a substance having volatility in a liquid state. In the spraying composition, component (a) is discharged from the nozzle tip toward the skin after the spraying composition placed in the electric field is sufficiently charged, and when component (a) evaporates, the charge density of the spraying composition becomes excessive, and component (a) evaporates further while being further refined by Coulomb repulsion, and component (a) is blended for the purpose of forming a finally dried coating on the skin. For this purpose, the volatile substance preferably has a vapor pressure at 20°C of 0.01 kPa or more and 106.66 kPa or less, more preferably 0.13 kPa or more and 66.66 kPa or less, further more preferably 0.67 kPa or more and 40.00 kPa or less, and even more preferably 1.33 kPa or more and 40.00 kPa or less.

Among the (a) volatile substances, for example, monovalent chain aliphatic alcohols, monovalent cyclic aliphatic alcohols, and monovalent aromatic alcohols, are suitably used as alcohols. Examples of the monovalent chain aliphatic alcohol include C1-C6 alcohol, the monovalent cyclic aliphatic alcohol includes C4-C6 cyclic alcohol, and the monovalent aromatic alcohol includes benzyl alcohol, and phenylethyl alcohol. Specific examples thereof include ethanol, isopropyl alcohol, butyl alcohol, phenylethyl alcohol, n-propanol, and n-pentanol. One or more of these alcohols can be used.

Among the (a) volatile substances, examples of the ketone include a C1-C4 dialkyl ketone, for example, acetone, methyl ethyl ketone, methyl isobutyl ketone. These ketones can be used alone or in combination of two or more.

The (a) volatile substance is more preferably one or more selected from the group consisting of ethanol, isopropyl alcohol, butyl alcohol and water, more preferably one or more selected from the group consisting of ethanol and butyl alcohol, and even more preferably a volatile substance at least containing ethanol.

The content of component (a) in the spraying composition is preferably 30 mass% or more, more preferably 55 mass% or more, and even more preferably 60 mass% or more. It is also preferably 98 mass% or less, more preferable 96 mass% or less, and even more preferably 94 mass% or less. The content of component (a) in the spraying composition is preferably 30 mass% or more and 98 mass% or less, more preferably 55 mass% or more and 96 mass% or less, and even more preferably 60 mass% or more and 94 mass% or less. By containing component (a) in the spraying composition at this ratio, the component (a) can be sufficiently volatilized and a coating on which powder is supported can be formed, when the electrostatic spraying method is performed.

The content of ethanol is preferably 50 mass% or more, more preferably 65 mass% or more, and even more preferably 80 mass% or more, based on the total amount of the (a) volatile substance. It is preferable that the content be 100 mass% or less. The content of ethanol is preferably 50 mass% or more and 100 mass% or less, more preferably 65 mass% or more and 100 mass% or less, and even more preferably 80 mass% or more and 100 mass% or less, based on the total amount of the (a) volatile substance.

The (b) polymer having a film-forming ability represents generally a substance which can be dissolved in the (a) volatile substance. Here, "be dissolved" means a dispersed state at 20°C, and the dispersed state means a visually uniform state, preferably a visually transparent or translucent state.

As the polymer having a film-forming ability, a polymer suitable for the nature of the volatile substance of the (a) volatile component may be used. Specifically, a polymer having a film-forming ability is roughly classified into a water-soluble polymer and a water-insoluble polymer. As used herein, the term "water-solublepolymer" means a polymer having a property in which, after weighing the polymer 1 g under the atmosphere of 1 atm and at 23°C, the polymer is immersed in 10 g of ion-exchanged water, and 0.5 g or more of the immersed polymer is dissolved in water after a lapse of 24 hours. The "water-insoluble polymer" in the present specification refers to a polymer having a property that 0.5 g or more of the polymer immersed in 10 g of ion-exchanged water is not dissolved after 24 hours after weighing the polymer 1 g under the atmosphere of 1 atm and 23°C.

Examples of the water-soluble polymer having a film-forming ability include a mucopolysaccharide such as pullulan, hyaluronic acid, chondroitin sulfate, poly-γ-glutamic acid, modified corn starch, β-glucan, a gluco-oligosaccharide, heparin, and keratosulfuric acid; a natural polymer such as cellulose, pectin, xylan, lignin, glucomannan, galacturonic acid, psyllium seed gum, tamarind seed gum, gum arabic, tragacanth gum, a soywater-soluble polysaccharide, alginic acid, carrageenan, laminan, agar (agarose), fucoidan, methylcellulose, hydroxypropyl cellulose, and hydroxy propyl methylcellulose; a synthetic polymer such as a partially saponified polyvinyul alcohol (not used in combination with a crosslinking agent), a low saponified polyvinyl alcohol, polyvinyl pyrrolidone (PVP), polyethylene oxide, and sodium polyacrylate. These water-soluble polymers can be used alone or in combination of two or more. Among these water-soluble polymers, pullulan, anda synthetic polymer such as a partially saponified polyvinyl alcohol, a low saponified polyvinyl alcohol, polyvinyl pyrrolidone, and polyethylene oxide are preferably used from the viewpoint of easiness in production of the coating. When polyethylene oxide is used as the water- soluble polymer, its number average molecular weight is preferably 50,000 or more and 3,000,000 or less, more preferably 100,000 or more and 2,500,000 or less.

Meanwhile, examples of the water-insoluble polymer having a film-forming ability include, a fully saponified polyvinyl alcohol which can be insolubilized after the formation of a coating, a partially saponified polyvinyl alcohol which can be crosslinked after the formation of a coating by using in combination with a crosslinking agent, an oxazoline-modified silicone such as a poly(N-propanoylethyleneimine)graft-dimethylsiloxane/γ-aminopropyl methylsiloxane copolymer, polyvinyl acetal diethyleminoacetate, Zein (a main component of corn protein), polyester, polylactic acid (PLA), an acrylic resin (e.g., a polyacrylonitrile resin, a polymethacrylic acid resin), a polystyrene resin, a polyvinyl butyral resin, a polyethylene terephthalate resin, a polybutylene terephthalate resin, a polyurethane resin, a polyamide resin, a polyimide resin, and a polyamide-imide resin. These water-insoluble polymers can be used alone or in combination of two or more. Among these water-insoluble polymers, it is preferable to use one or more selected from the group consisting of a fully saponified polyvinyl alcohol which can be insolubilized after the formation of a coating, a partially saponified polyvinyl alcohol which can be crosslinked after the formation of a coating by using in combination with a crosslinking agent, a polyvinyl butyral resin, a polyurethane resin, an oxazoline-modified silicone such as a poly(N-propanoylethyleneimine) graft-dimethylsiloxane/γ-aminopropylmethylsiloxane copolymer, water-soluble polyester and Zein, and it is more preferable to use one or more selected from the group consisting of a polyvinyl butyral resin and a polyurethane resin.

The content of component (b) in the spraying composition is preferably 2 mass% or more, more preferably 4 mass% or more, and even more preferably 6 mass% or more. It is also preferably 50 mass% or less, more preferably 45 mass% or less, and even more preferably 40 mass% or less. The content of component (b) in the spraying composition is preferably 2 mass% or more and 50 mass% or less, more preferably 4 mass % or more and 45 mass% or less, and even more preferably 6 mass% or more and 40 mass% or less. By blending component (b) in the spraying composition at this ratio, a desired coating can be efficiently formed.

From the viewpoint that when the electrostatic spraying method is performed, component (a) is sufficiently volatized to form a coating on the skin without secondary powder aggregation, and as a result, the electrostatic spraying can form a coating which uniformly sticks to skin hills, skin grooves and skin pores after application to the skin, and in terms of preventing color unevenness, white float and makeup collapse, the content ratio of component (a) to component (b) in the spraying composition, ((a)/(b)), is preferably 0.5 or more and 40 or less, more preferably 1 or more and 30 or less, and even more preferably 2 or more and 25 or less.

In addition, from the viewpoint that when the electrostatic spraying method is performed, ethanol (a) is sufficiently volatized to form a coating on the skin without secondary powder aggregation, and as a result, the electrostatic spraying can form a coating which uniformly sticks to skin hills, skin grooves and skin pores after application to the skin, and in terms of preventing color unevenness, white float and makeup collapse, the content ratio of ethanol (a) to component (b) in the spraying composition, ((a)/(b)), is preferably 0.5 or more and 40 or less, more preferably 1 or more and 30 or less, and even more preferably 2 or more and 25 or less.

The (c) powder is a powder which can be formulated into a cosmetic, and is formulated for the purpose of forming a cosmetic coating on the skin. Such powders include a coloring pigment, a constitutive pigment, a pearl pigment, an organic powder, and the like. The coloring pigment includes an inorganic coloring pigment, an organic coloring pigment, and an organic dye, and one or more of these can be used.

Specific examples of the inorganic coloring pigment include an inorganic color pigment such as red iron oxide, iron hydroxide, iron titanate, yellow iron oxide, black iron oxide, carbon black, dark blue, ultramarine blue, dark blue titanium oxide, black titanium oxide, a sintered product of titanium and titanium oxide, manganese violet, cobalt violet, chromium oxide, chromium hydroxide, cobalt oxide, cobalt titanate; an inorganic white pigment such as titanium oxide, zinc oxide, calamin, zirconium oxide, magnesium oxide, cerium oxide, aluminum oxide, and a composite thereof. One or more of these can be used.

Of these, at least one or more selected from the group consisting of iron oxide, titanium oxide, and zinc oxide are preferable, and one or more selected from the group consisting of titanium oxide, zinc oxide, red iron oxide, yellow iron oxide, and black iron oxide are more preferable.

Examples of the organic coloring pigment and the organic dye include organic tar pigments such as red No. 3, red No. 102, red No. 104, red No. 106, red No. 201, red No. 202, red No. 204, red No. 205, red No. 220, red No. 226, red No. 227, red No. 228, red No. 230, red No. 401, red No. 405, red No. 505, orange No. 203, orange No. 204, orange No. 205, yellow No. 4, yellow No. 5, yellow No. 401, blue No. 1, and blue No. 404; and organic dyes such as β-carotene, caramel, and paprika dyes. In addition, apolymer coated with cellulose, polymethacrylic acid ester, or the like may be used. Of these, it is preferable to contain at least red No. 102.

Examples of the constitutive pigment include barium sulfate, calcium sulfate, magnesium sulfate, magnesium carbonate, calcium carbonate, talc, mica, kaolin, sericite, silicic acid, anhydrous silicic acid, aluminum silicate, magnesium silicate, magnesium aluminum silicate, calcium silicate, barium silicate, strontium silicate, metal tungstate, hydroxyapatite, vermiculite, clay, bentonite, montmorillonite, hectolite, smectite, zeolite, ceramic powder, dibasic calcium phosphate, alumina, silica, aluminum hydroxide, boron nitride, synthetic mica, synthetic sericite, metal soap, barium sulfate-treated mica, and the like. One or more of these can be used.

Among these, barium sulfate, calcium carbonate, mica, anhydrous silicic acid, talc, boron nitride, and synthetic mica are preferably included.

Examples of the pearl pigment (glittering powders) include fish foil, titanium oxide-coated mica (mica titanium), bismuth oxychloride, titanium oxide-coated bismuth oxychloride, titanium oxide-coated talc, titanium oxide-coated color mica, (titanium oxide/iron oxide)-coated mica, microparticulate titanium oxide-coated mica titanium, microparticulate zinc oxide-coated mica titanium, organic pigment-treated mica titanium, low-order titanium oxide-coated mica, titanium oxide-coated synthetic mica, titanium oxide-coated platy silica, hollow plate-like titanium oxide, iron oxide-coated mica, platy iron oxide (MIO), an aluminum flake, a stainless flake, titanium oxide-coated plate alumina, a glass flake, a titanium oxide-coated glass flake, pearl shell, gold foil, a gold vapor-coated resin film, and a metal vapor-coated resin film. One or more thereof can be used.

Examples of the organic powder include a silicone rubber powder, a silicone resin-coated silicone rubber powder, polymethylsilsesquioxane, a polyamide powder, a nylon powder, a polyester powder, a polypropylene powder, a polystyrene powder, a polyurethane powder, a vinyl resin powder, a urea resin powder, aphenolic resin powder, a fluorine resin powder, a silicon resin powder, an acrylic resin powder, a melamine resin powder, a polycarbonate resin, a divinylbenzene-styrene copolymer, a silk powder, a wool powder, a cellulose powder, a long-chain alkyl phosphoric acid metal salt, an N-mono long-chain alkyl acyl basic amino acid, and a composite thereof. One or more of these can be used.

Among these, it is preferable to contain a cellulose powder, a silicone rubber powder, a silicone resin-coated silicone rubber powder, polymethylsilsesquioxane, an acrylic resin powder, or a nylon powder.

Any of the powders used in the present invention can be used as it is, and one or more of them can be used after subjected to hydrophobic treatment. The hydrophobic treatment is not limited as long as it is applied to usual cosmetic powders, and dry treatment, wet treatment, or the like may be performed using a surface treatment agent such as a fluorine-based compound, a silicone-based compound, a metal soap, an amino acid compound, lecithin, an alkylsilane, an oil agent, or an organic titanate.

Specific examples of the surface treatment agent include: a fluorine-based compound such as a perfluoropolyether, a perfluoroalkyl phosphate ester, a perfluoroalkyl alkoxysilane, a fluorine-modified silicone; a silicone-based compound such as a dimethylpolysiloxane, a cyclic silicone, a single-terminal or a double-terminal trialkoxy group-modified organopolysiloxane, a crosslinked silicone, a silicone resin, a fluorine-modified silicone resin, and an acryl-modified silicone; a metal soap such as aluminum stearate, aluminum myristate, zinc stearate, and magnesium stearate; an amino acid compound such as proline, hydroxyproline, alanine, glycine, sarcosine, glutamic acid, aspartic acid, lysine and a derivative thereof; lecithin, hydrogenated lecithin; an alkyl silane such as methyltrimethoxysilane, ethyltrimethoxysilane, hexyltrimethoxysilane, octyltrimethoxysilane, and octyltriethoxysilane; an oil agent such as polyisobutylene, wax and a fat; and an organic titanate such as isopropyltitanium triisostearate.

As the powder used in the present invention, one or more of them may be further subjected to hydrophilic treatment. The hydrophilic treatment is not limited as long as it is applied to a normal cosmetic powder.

For example, examples of the powder include a plant-based polymer such as gum arabia, tragacanth, arabinogalactan, locust bean gum (carob gum), guar gum, karaya gum, carrageenan, pectin, agar, quince seed (marmelo), starch (rice, corn, pepper, wheat), algelloid, trancho gum, and locust bean gum; a microbial-based polymer such as xanthan gum, dextran, succinoglucan, and pullulan; and an animal-based polymer such as collagen, casein, albumin, deoxyribonucleic acid (DNA) and a salt thereof; a starch-based polymer such as a carboxymethyl starch, and a methyl hydroxypropyl starch; a cellulose-based polymer such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and apowdered cellulose ; an alginic acid-based polymer such as sodium alginate and a propylene glycol alginate ester; a vinyl polymer such as polyvinyl methyl ether, polyvinyl pyrrolidone and a carboxyvinyl polymer; a polyoxyethylene polymer such as polyethylene glycol and polyethylene glycol silane; a polyoxyethylene polyoxypropylene copolymer-based polymer; an acrylic polymer such as sodium polyacrylate, polyethyl acrylate and polyacrylic acid amide; and an inorganic silicic acid compound such as silica.

As the powder, as long as it is a powder normally used in cosmetics, a powder having a shape such as a sphere, a plate, a needle, or an amorphous shape, a particle diameter such as a fume shape, a fine particle shape, or a pigment grade, a particle structure such as porous or non-porous, or the like can be used.

The average particle diameter of the (c) powder is preferably 0.001 µm or more and 200 µm or less, more preferably 0.01 µm or more and 50 µm or less, further more preferably 0.02 µm or more and 20 µm or less, and even more preferably 0.05 µm or more and 10 µm or less, from the viewpoint that the powder adheres uniformly to the hills, skin grooves and skin pores and provides a natural cosmetic feeling.

In the present invention, the average particle size of the powder is measured by electron-microscopy, laser diffraction/scattering particle size distributor. Specifically, for the laser diffraction/scattering method, ethanol is used as the dispersion medium and measured by a laser diffractive particle size distribution meter (e.g., Seishin Corporation, LMS-350) . When the powder is subjected to hydrophobic treatment or hydrophilic treatment, the average particle diameter and the content of component (c) mean the average particle diameter and the mass including the hydrophobic treatment or the hydrophilic treatment agent.

Component (c) can be one or more types, from the viewpoint of preventing secondary aggregation of the powder by supporting the powder in the coating and uniformly sticking to skin hills, skin grooves and skin pores even after application to the skin, and preventing color unevenness, white float, and cosmetic fading, the content is preferably 0.001 mass% or more, more preferably 0.01 mass% or more, further more preferably 0.1 mass% or more; preferably 50 mass% or less, more preferably 30 mass% or less, and even more preferably 15 mass% or less in the spraying composition. The content of the component (c) is preferably 0.001 mass% or more and 50 mass% or less, more preferably 0.01 mass% or more and 30 mass% or less, and even more preferably 0.1 mass% or more and 15 mass% or less.

The mass ratio of the coloring pigment to the total powder (component (c)), (coloring pigment/component (c)), is preferably 0.2 or more, more preferably 0 . 3 or more, even more preferably 0.4 or more; and preferably 1. 0 or less from the viewpoint of preventing color unevenness and white float.

The content ratio of component (c) to component (b) in the spraying composition, ((c)/(b)), is preferably 0.001 or more and 20 or less, more preferably 0.002 or more and 1 or less, and even more preferably 0.005 or more and 0.5 or less, from the viewpoint of suppressing the secondary aggregation of the powder in the spraying composition, uniformly adhering to the skin hills, skin grooves and skin pores, and preventing color unevenness, white float, and cosmetic kindness even after application to the skin.

In addition, the present invention may include an oil agent in the spraying composition. The oil agent is an oil which can be formulated in cosmetics and are formulated for the purpose of forming a uniform cosmetic coating containing components (b) and (c) on the skin.

As the oil agent, a polar oil in a liquid state at 20°C can be preferably used, and a hydrocarbon oil, an ester oil, an ether oil, a higher alcohol, and a silicone oil can be included, and one or more kinds of oils can be used in combination.

Examples of the hydrocarbon oil include liquid paraffin, squalane, squalene, liquid isoparaffin, n-octane, n-heptane, cyclohexane, light isoparaffin, and the like, and liquid paraffin and squalane are preferable from the viewpoint of impression from use and adhesivity. In addition, isododecane, isohexadecane, or hydrogenated polyisobutene having a viscosity of less than 10 mPa·s at 30°C is preferable from the viewpoint of uniformly adhering the electrostatically sprayed coating to the skin and suppressing color unevenness.

The viscosity here is measured at 30°C with a B-type viscometer (Model TVB-10, manufactured by Tokimek Corporation, measurement conditions: Rotor No.1, 60 rpm, 1 minute).

The content of the hydrocarbon oil is preferably 0.01 mass% or more, more preferably 0.1 mass% or more, and preferably 10 mass% or less, more preferably 5 mass% or less, and even more preferably 2 mass% or less in the spraying composition, from the viewpoint of uniformly adhering the electrostatically sprayed coating to the skin and suppressing color unevenness.

Examples of the ester oil include an ester consisting of a linear or branched chain fatty acid and a linear or branched chain alcohol or a polyhydric alcohol. Specifically, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethyleneglycol di(2-ethylhexanoate), dipentaerythritol fatty acid ester, N-alkylglycol monoisostearate, neopentylglycol dicaprate, diisostearyl malate, glyceryl di(2-heptylundecanoate), trimethylolpropane tri(2-ethylhexanoate), trimethylolpropane triisostearate, pentaerythritol tetra(2-ethylhexanoate), glyceryl tri(2-ethylhexanoate), trimethylolpropane triisostearate, cetyl 2-ethylhexanoate, 2-ethylhexyl palmitate, diethylhexyl naphthalenedicarboxylate, (C12-15)alkyl benzoate, cetearyl isononanoate, caprylic/capric triglyceride, dicaprylic/capric butyleneglycol, glyceryl trilaurate, glyceryl trimyristate, glyceryl tripalmitate, glyceryl triisostearate, glyceryl tri(2-heptylundecanoate), glyceryl tribehenate, glyceryl tricocoate, castor oil fatty acid methyl ester, oleyl oleate, 2-heptylundecyl palmitate, diisobutyl adipate, N-lauryol-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di(2-ethylhexyl) succinate, triethyl citrate, 2-ethylhexyl para-methoxycinnamate, tripropylene glycol dipivalate, di(phytosteryl/octyldodecyl) lauroyl glutamate, dipentaerythrityl tri-polyhydroxystearate, dipentaerythrityl pentaisostearate, dipentaerythrityl tetraisostearate, pentaerythrityl (behenic acid/polyhydroxystearate), an olive oil, a jojoba oil, a macadamia nut oil, a medoform oil, a castor oil, a safflower oil, a sunflower oil, an avocado oil, a canola oil, an apricot kernel oil, a rice germ oil, a rice bran oil.

Among them, from the viewpoint of pasting the electrostatic sprayed coating over the skin and excellent feel when applied to the skin, at least one selected from the group consisting of octyldodecyl myristate, myristyl myristate, isocetyl stearate, isocetyl isostearate, cetearyl isononanoate, diisobutyl adipate, di-ethylhexyl cebacate, isopropyl myristate, isopropyl palmitate, diisostearyl malate, neopentyl glycol dicaprylate, (C12-15)alkyl benzoate, caprylic/capric triglyceride, di(phytosteryl/octyldodecyl) lauroyl glutamate, dipentaerythrityl tri-polyhydroxystearate dipentaerythrityl pentaisostearate, dipentaerythrityl tetraisostearate, and pentaerythrityl (behenic acid/polyhydroxystearic acid) are preferred. At least one selected from the group consisting of isopropyl myristate, isopropyl palmitate, diisostearyl malate, neopentyl glycol dicaprate, (C12-15) alkyl benzoate, (caprylic/capric) triglyceride, di(phytosteryl/octyldodecyl) lauroyl glutamate , dipentaerythrityl tri-polyhydroxystearate, dipentaerythrityl pentyisostearate, dipentaerythrityl tetraisostearate, and pentaerythrityl (behenic acid/polyhydroxystearic acid), are more preferred. At least one selected from the group consisting of neopentyl glycol dicaprylate, diisostearyl malate, (caprylic /capric) triglyceride, and di (phytosteryl/octyldodecyl) lauroyl glutamate are even more preferred.

The content of the ester oil is preferably 0.01 mass% or more, more preferably 0.1 mass% or more; preferably 10 mass% or less, more preferably 5 mass% or less, even more preferably 2 mass% or less in the spraying composition, from the viewpoint of uniformly adhering the electrostatically sprayed coating to the skin and suppressing color unevenness.

Examples of the ether oil include cetyl-1,3-dimethylbutyl ether, dicapryl ether, dilauryl ether, diisostearyl ether.

The content of the ether oil is preferably 0.01 mass% or more, more preferably 0.1 mass% or more; preferably 10 mass% or less, preferably 5 mass% or less, more preferably 2 mass% or less in the spraying composition, from the viewpoint of uniformly adhering the electrostatically sprayed coating to the skin and suppressing color unevenness.

Examples of the higher alcohol include a liquid higher alcohol having 12 to 20 carbon atoms, and branched or unsaturated higher alcohols are preferable, specifically isostearyl alcohol, oleyl alcohol, octyldodecanol, and the like.

The content of the higher alcohol is preferably 0.01 mass% or more, more preferably 0.1 mass% or more; preferably 10 mass% or less, more preferably 5 mass% or less, even more preferably 2 mass% or less in the spraying composition, from the viewpoint of uniformly adhering the electrostatically sprayed coating to the skin and suppressing color unevenness.

Examples of the silicone oil include dimethylpolysiloxane, dimethylcyclopolysiloxane, methylphenylpolysiloxane, methylhydrogenpolysiloxane, higher alcohol modified organopolysiloxane, and preferably include at least dimethylpolysiloxane, from the viewpoint of adhering the electrostatically sprayed coating to the skin.

The kinematic viscosity of the silicone oil at 25°C is preferable 3 mm² per second or more, more preferably 4 mm² per second or more, even more preferably 5 mm² per second or more; preferably 30 mm² per second or less, more preferably 20 mm² per second or less, and even more preferably 10 mm² per second or less from the viewpoint of adhering the electrostatically sprayed coating to the skin.

The content of the silicone oil is preferably 0.01 mass% or more, more preferably 0.1 mass% or more; preferably 10 mass% or less, more preferably 5 mass% or less, and even more preferably 2 mass% or less in the spraying composition, from the viewpoint of uniformly adhering the electrostatically sprayed coating to the skin and suppressing color unevenness.

The content of the oil agent is preferably 0.01 mass% or more, more preferably 0.1 mass% or more; preferably 30 mass% or less, more preferably 20 mass% or less, and even more preferably 10 mass% or less in the spraying composition, from the viewpoint of uniformly adhering the electrostatically sprayed coating to the skin and suppressing color unevenness.

In addition, the spraying composition may contain a glycol. Examples of the glycol include ethylene glycol, propylene glycol, butylene glycol, diethylene glycol, dipropylene glycol, polypropylene glycol, and the like. In order to sufficiently volatilize component (a) when the electrostatic spraying method is performed and to suppress the secondary aggregation of the powder, the content of glycol is preferably 10 mass% or less, more preferably 3 mass% or less, further more preferably 1 mass% or less, and it is also preferable that substantially no glycol be contained in the spraying composition.

The spraying composition may contain only the above-mentioned components (a), (b) and (c), or may contain other components in addition to components (a), (b) and (c). Examples of the other components include a plasticizer of the (b) polymer having a film-forming ability, a surfactant, a perfume, a repellent, an antioxidant, a stabilizer, a preservative, various vitamins, and the like, in addition to the oil agent.

When other components are included in the spraying composition, the content ratio of the other components is preferably 0.1 mass% or more and 30 mass% or less, and more preferably 0.5 mass% or more and 20 mass% or less.

The present invention is characterized in that the spraying compositions are electrostatically sprayed directly onto the skin.

When the electrostatic spraying method is performed, a composition having a viscosity at 25°C of 1 mPa·s or more, preferably 10 mPa·s or more, more preferably 50 mPa·s or more is usedas the spraying composition. In addition, a material having a viscosity at 25°C of 5,000 mPa·s or less, more preferably 2,000 mPa·s or less, and even more preferably 1,500 mPa·s or less is used. The viscosity of the spraying composition at 25°C is preferably 1 mPa·s or more and 5,000 mPa·s or less, more preferably 10 mPa·s or more and 2,000 mPa·s or less, and further more preferably 50 mPa·s or more and 1,500 mPa·s or less. By using a spraying composition having a viscosity in this range, it is possible to successfully form a porous coating comprising a powder, in particular a porous coating consisting of a deposit of fibers comprising the powder, by means of an electrostatic spraying process. The formation of the porous coating containing the powder is advantageous from the viewpoints of uniform stickability of the coating containing the powder to the skin hills, skin grooves and skin pores, prevention of color unevenness, prevention of white float, prevention of cosmetic kinking, adhesivity of the coating, and the like. The viscosity of the spraying composition is measured at 25° C using a type E viscometer. As the E-type viscometer, for example, an E-type viscometer manufactured by Tokyo Meters Corporation can be used. In this case, the rotor No.43 can be used as the rotor.

The spraying composition is sprayed directly onto the area of the human skin to provide makeup of the powder by electrostatic spraying. Electrostatic spraying involves electrostatically spraying the spraying composition onto the skin using an electrostatic spraying device. An electrostatic spraying device basically includes a container containing the composition, a nozzle for discharging the composition, a supply device for supplying the composition contained in the container to the nozzle, and a power supply for applying a voltage to the nozzle.

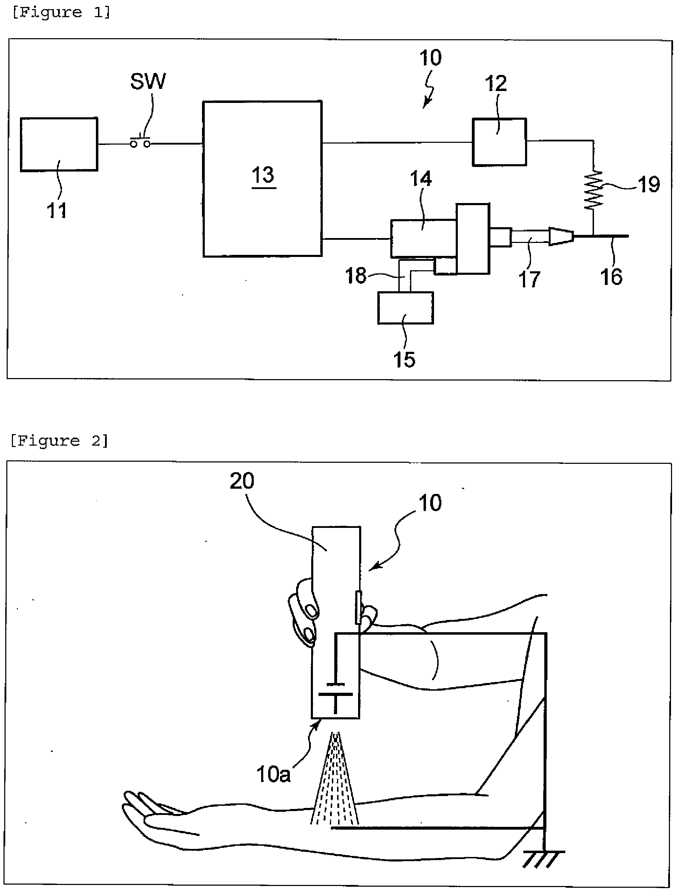

FIG. 1 is a schematic diagram showing a configuration of an electrostatic spraying device preferably used in the present invention. The electrostatic spraying device 10 shown in the figure includes a low-voltage power supply 11. The low-voltage power supply 11 can generate a voltage of several volts and below 20 volts. For the purpose of enhancing the portability of the electrostatic spraying device 10, the low-voltage power supply 11 preferably comprises one or more batteries. Further, by using a battery as the low-voltage power supply 11, there is an advantage that replacement can be easily performed as necessary. Instead of the battery, an AC adapter or the like can be used as the low-voltage power supply 11.

The electrostatic spraying device 10 also includes a high-voltage power supply 12. The high-voltage power supply 12 is connected to the low-voltage power supply 11, and includes an electric circuit (not shown) for boosting a voltage generated by the low-voltage power supply 11 to a high voltage. The booster electric circuit is generally composed of a transformer, a capacitor, a semiconductor element, and the like.

The electrostatic spraying device 10 further comprises an auxiliary electrical circuit 13. The auxiliary electric circuit 13 is interposed between the low-voltage power supply 11 and the high-voltage power supply 12, and has a function of adjusting the voltage of the low-voltage power supply 11 to stably operate the high-voltage power supply 12. Further, the auxiliary electric circuit 13 has a function of controlling the number of revolutions of a motor provided in a microgear pump 14, which will be described hereafter. By controlling the rotation speed of the motor, the supply amount of the spraying composition from the container 15 of the spraying composition described later to the microgear pump 14 is controlled. A switch SW is mounted between the auxiliary electric circuit 13 and the low-voltage power supply 11 so that the electrostatic spraying device 10 can be turned on and off by turning the switch SW on and off.

The electrostatic spraying device 10 further comprises a nozzle 16. The nozzle 16 is made of a conductive material such as metal or a non-conductive material such as plastic, rubber, ceramic, or the like, and has a shape capable of discharging the spraying composition from the tip thereof. A microspace through which the spraying composition flows is formed in the nozzle 16 along the longitudinal direction of the nozzle 16. The size of the cross section of the microspace is preferably 100 µm or more and 1,000 µm or less in terms of diameter.

The nozzle 16 communicates with the microgear pump 14 via a pipe 17. The pipe 17 may be conductive or non-conductive. The nozzle 16 is electrically connected to the high-voltage power supply 12. This makes it possible to apply a high voltage to the nozzle 16. In this case, in order to prevent an excessive current from flowing when a human body directly touches the nozzle 16, the nozzle 16 and the high-voltage power supply 12 are electrically connected via a current limiting resistor 19.

The microgear pump 14, which communicates with the nozzle 16 via a pipe 17, functions as a supply device for supplying the nozzle 16 with the spraying composition contained in the container 15. The microgear pump 14 is operated by receiving a power supply from the low-voltage power supply 11. The microgear pump 14 is configured to supply a predetermined amount of the spraying composition to the nozzle 16 under the control of the auxiliary electric circuit 13.

A container 15 is connected to the microgear pump 14 via a flexible pipe 18. The container 15 contains a spraying composition. The container 15 preferably has a cartridge-type replaceable configuration.

The electrostatic spraying device 10 having the above configuration can be used, for example, as shown in FIG. 2. FIG. 2 shows an electrostatic spraying device 10 of the hand-held type which is dimensioned to be holdable with one hand. The electrostatic spraying device 10 shown in the same figure contains all of the members of the configuration diagram shown in Fig. 1 in the cylindrical housing 20. A nozzle, not shown, is disposed at one longitudinal end 10a of the housing 20. The nozzle is disposed in the housing 20 such that the blowing direction of the composition coincides with the longitudinal direction of the housing 20 and is convex toward the skin side. By arranging the nozzle tip so as to be convex toward the skin in the longitudinal direction of the housing 20, the spraying composition hardly sticks to the housing, and the coating can be stably formed.

When the user intends to operate the electrostatic spraying device 10, the user, i.e., the person who forms the coating on the area on the skin to be made of powder by electrostatic spraying, holds the device 10 by hand and directs one end 10a of the device 10, on which a nozzle, not shown, is arranged, to the application site where the electrostatic spraying is to be performed. In FIG. 2, one end 10a of the electrostatic spraying device 10 is shown pointing inside the user' s forearm. In this state, the device 10 is switched on to perform the electrostatic spraying method. When the device 10 is powered on, an electric field is generated between the nozzle and the skin. In the embodiment shown in FIG. 2, a positive high voltage is applied to the nozzle and the skin becomes the negative electrode. When an electric field is generated between the nozzle and the skin, the spraying composition at the tip of the nozzle is polarized by electrostatic induction so that the tip portion becomes conical, and droplets of the spraying composition charged from the tip of the cone are discharged into the air toward the skin along the electric field. When component (a) as a solvent evaporates from the spraying composition discharged into the space and charged, the charge density on the surface of the spraying composition becomes excessive, and spreads into the space by repeated refinement by Coulomb repulsion force, and reaches the skin. In this case, by appropriately adjusting the viscosity of the spraying composition, the sprayed composition can reach the application site in the form of droplets. Alternatively, while the spraying composition is discharged to a space, it is also possible to deposit a fiber formed by stretch deformation by an electric potential difference onto an application site, with a volatile substance (solvent) being evaporated out of the droplet and a film-forming polymer (solute) being solidified. For example, increasing the viscosity of the spraying composition facilitates deposition of the composition in the form of fibers at the site of application. This forms a porous coating of a deposit of fibers on the surface of the application site. The porous coating of the deposit of fibers containing powder can also be formed by adjusting the distance between the nozzle and the skin and the voltage applied to the nozzle.

During electrostatic spraying, a high potential difference occurs between the nozzle and the skin. However, since the impedance is very large, the current flowing through the human body is extremely small. For example, the present invention has confirmed that the current flowing through the human body during the electrostatic spraying process is several orders of magnitude smaller than the current flowing through the human body due to static electricity generated during normal living.

When a fiber deposit containing powder is formed by an electrostatic spraying method, the thickness of the fiber is preferably 10 nm or more, more preferably 50 nm or more, when expressed as a circle equivalent diameter. The thickness is preferably 3,000 nm or less, and more preferably 1500 nm or less. The thickness of the fiber can be measured by observing the fiber at a magnification of 10,000 by, for example, scanning electron microscopy (SEM) observation, removing defects (clumps of fibers, crossing portions of fibers, and droplets) from the two-dimensional image, arbitrarily selecting 10 fibers, drawing a line orthogonal to the longitudinal direction of the fiber, and directly reading the fiber diameter.

The coating, which is a deposit of fibers formed by electrostatic spraying, has a powder-bearing coating in which component (c) is present inside the constituent fibers. By the inside of the fiber is meant an inclusion or a portion of a surface. If the content of component (c) is approximately 0.001 mass% or more and 50 mass% or less, the adhesivity of the constituent fiber to the skin is high and a powder-bearing coating excellent in the following property to the skin is easily formed although it depends on the affinity between the polymer and component (c), whereas if the content ratio of component (c) in the spraying composition is more than 50 mass%, the powder-bearing coating is hardly formed on the inner side of the constituent fiber. When the powder-bearing coating is formed on the fiber constituting the coating in this manner, the coating tends to become translucent because the powder is covered by the fiber, and approaches a natural appearance having a soft focus effect. Furthermore, increase in the adhesion durability serves to maintaining the makeup finish. The effect of the adhesivity and the effect of the uniformity of the cosmetic effect by the powder are further enhanced by the application of a liquid agent described later.

The contents of components (a), (b) and (c) in the spraying composition are measured as follows. Since component (a) which is a volatile substance does not exist in the formed film or volatilizes even if it exists, the formed film is measured in a state in which only component (b) and component (c) are contained, and the content thereof is measured as follows.

### <Determination of the Content of Components (a), (b) and (c) in the spraying composition>

Separation/identification by liquid chromatography (HPLC) and identification by infrared spectrophotometer (IR) are available in solution. Since the liquid chromatograph elutes at first a component having a large molecular weight, the composition can be identified by predicting the molecular weight or by determining the elution position of component. It is also possible to assign and identify functional groups from individual absorbers by IR analysis, and it is generally possible to identify them by comparing IR charts of components with standard charts of commercial additives.

### <Determination of the Contents of Components (b) and (c) in the formed coating>

A solvent capable of dissolving the coating is searched for, and after dissolving the coating in the solvent, separation/identification by a liquid chromatograph (HPLC) or identification by an infrared spectrophotometer (IR) is performed.

The fiber forming the coating is a continuous fiber of infinite length in principle of production, but it is preferable that the fiber have a length of at least 100 times or more of the thickness of the fiber. In the present specification, a fiber having a length of 100 times or more of the thickness of the fiber is defined as a "continuous fiber". The coating produced by the electrostatic spraying method is preferably a porous discontinuous coating composed of a deposit of continuous fibers. Such a form of coating not only can be handled as a single sheet as an aggregate, but also has the advantage that it has a very soft feature, and it is difficult to be separated even when a shearing force is applied to it, and it is excellent in the following property to the movement of the body. In addition, there is an advantage that the coating can be easily completely removed. Meanwhile, a continuous coating having no pores is not easy to peel off and has a low sweat dissipation property, so that there is a fear that the skin will become blistered. In addition, it is difficult to completely remove the porous discontinuous coating formed of the aggregate of particles without damaging the skin, for example, an operation of applying friction or the like to the entire coating is necessary in order to completely remove the coating.

In the electrostatic spraying process using the electrostatic spraying device 10, the spraying composition which has been electrostatically sprayed into a fibrous state directly reaches the skin in a state in which component (b) and component (c) are charged while component (a) evaporates. Since the skin is also charged as described above, the fibers adhere to the skin in the form of a single membrane by electrostatic forces. Since fine irregularities such as texture are formed on the surface of the skin, the fibers are more closely adhered to the surface of the skin in the form of a single membrane in combination with the anchoring effect due to the irregularities. When the electrostatic spraying is completed in this manner, the power of the electrostatic spraying device 10 is turned off. As a result, the electric field between the nozzle and the skin disappears, and the surface of the skin is immobilized with an electric charge. Consequently, the adhesivity of the coating in the form of a single film is further developed, making it difficult to peel the coating from its edge during wearing, and durability during use is improved. In addition, since the fiber constituting the coating contains component (c), not only an excellent cosmetic effect can be obtained, but the presence of component (c) in the fiber enhances the anchoring effect on the fine concavo-convex surface of the skin, so that the coating can be sufficiently adhered to the skin. The reason for this is considered to be that the presence of component (c) in the fiber enhances the following ability of the skin to a fine concavo-convex surface . Furthermore, since the powder-bearing coating has component (c) inside the fibers constituting the coating, the light is easily scattered, and the coating can cover the skin in a natural appearance due to the soft focus effect. These effects are further enhanced by a liquid agent application described later.

The distance between the nozzle and the skin also depends on the voltage applied to the nozzle, but 50 mm or more and 150 mm or less are preferred for successfully forming the coating. The distance between the nozzle and the skin can be measured by a commonly used non-contact sensor or the like.

Whether or not the coating formed by the electrostatic spraying method is porous, the basis weight of the coating is preferably 0.1 g/m² or more, more preferably 1 g/m² or more. Further, it is preferable to be 50 g/m² or less and more preferable to be 40 g/m² or less. For example, the basis weight of the coating is preferably 0.1 g/m² or more and 50 g/m² or less, and more preferably 1 g/m² or more and 40 g/m² or less. By setting the basis weight of the coating in this manner, the cosmetic effect of the powder and the adhesivity of the coating can be improved.

The electrostatic spraying step of forming a coating by electrostatically spraying the composition directly onto the skin means a step of electrostatically spraying onto the skin to form a coating. The process of electrostatically spraying the composition to a location other than the skin to produce a sheet of fibers and applying the sheet to the skin differs from the electrostatically sprayingprocess described above.

In the present invention, it is also possible to carry out, before or after the electrostatic spraying step of forming the coating by electrostatic spraying as described above, or before or after the electrostatic spraying step, a liquid agent application step of applying to the skin by a method other than electrostatic spraying a liquid agent which contains one or more of water, polyols and oils which are liquid at 20 °C. By applying the liquid agent prior to the electrostatic spraying step, the skin surface is more easily polarized by electrostatic induction, the sprayed spraying composition is more strongly stuck by electrostatic force, and the transparency of the appearance of the sprayed spraying composition can be improved. In addition, by performing the liquid agent application step by a method other than electrostatic spraying after the electrostatic spraying step, in particular, immediately after the electrostatic spraying step, the coating formed in the electrostatic spraying step becomes easily familiar to the skin with the liquid agent, and the coating adheres highly to the skin, and the transparency is further improved. A space is less likely to be made between the edge of the coating and the skin, which also improves the adhesivity between the coating and the skin. As a result, even if a coating is formed in a region where the degree of elongation and contraction of the skin is large or in a region where the curvature is large, peeling, tearing, or the like does not easily occur. Suitably, when the coating is a porous coating composed of a deposit of fibers, the adhesivity with the skin is high despite the high porosity, and a large capillary force is apt to occur. Further, when the fibers are fine, it is easy to make the porous coating have a high specific surface area.

Before and/or after the step of forming the porous coating consisting of a deposit of fibers in the electrostatic spraying step, a liquid agent application step is performed by a method other than electrostatic spraying to form a carrier coating of the humectant and the powder between the fibers forming the porous coating and/or on the fiber surface in which the humectant and the powder are present. This improves the adhesivity of the coating. Especially when the coating is colorless and transparent or translucent, it becomes more difficult to see the coating, thereby making it look like natural skin. In addition, when the coating is colored and opaque, the coating has a transparent feeling, so that it can appear as a part of the skin. The colored color includes white.

When the liquid agent used in the liquid agent application step contains water, the liquid agent includes, for example, water, an aqueous liquid such as an aqueous solution and an aqueous dispersion. In addition, cosmetic water, emulsions composed, emulsions such as O/W emulsions and W/O emulsions, cosmetic creams, aqueous liquids thickened with thickeners, and the like can also be included.

When the liquid agent used in the liquid agent application step contains a polyol, examples of the polyol include alkylene glycols such as ethylene glycol, propylene glycol, 1,3-propanediol, and 1,3-butanediol; polyalkylene glycols such as diethylene glycol, dipropylene glycol, polyethylene glycol, and polypropylene glycol; and glycerins such as glycerin, diglycerin, and triglycerin. Of these, ethylene glycol, propylene glycol, 1,3 -butanediol, dipropylene glycol, polyethylene glycol, glycerin, and diglycerin are preferable from the viewpoint of smoothness at the time of application, and further propylene glycol, 1, 3-butanediol, and glycerin are more preferable, and propylene glycol and 1,3-butanediol are even more preferable.

Meanwhile, when the liquid agent used in the liquid agent application step contains a liquid oil at 20°C (hereinafter also referred to as a "liquid oil"), the liquid oil at 20°C includes, for example, a linear or branched hydrocarbon oil such as liquid paraffin, light isoparaffin, liquid isoparaffin, squalane, and squalene; vegetable oils such as a jojoba oil, and an olive oil; animal oils such as a liquid lanolin, an ester oil such as a monoalcohol fatty acid ester, and a polyvalent alcohol fatty acid ester; and a silicone oil such as dimethylpolysiloxane, dimethylcyclopolysiloxane, methylphenylpolysiloxane, methylhydrogenpolysiloxane, and a higher alcohol modified organopolysiloxane. Among these oils, a hydrocarbon oil, an ester oil, a vegetable oil containing a triglyceride, or the like, a polar oil such as a silicone oil, or the like is preferable from the viewpoint of impression from use such as smoothness at the time of application, and a hydrocarbon oil, an ester oil, or a triglyceride is more preferable. In addition, one or more liquid oils selected from these can be used alone or in combination.

Examples of the hydrocarbon oil include liquid paraffin, squalane, squalene, n-octane, n-heptane, cyclohexane, light isoparaffin, liquid isoparaffin, and the like, and liquid paraffin and squalane are preferable from the viewpoint of impression from use. In addition, from the viewpoint of adhering the electrostatically sprayed coating to the skin, the viscosity of the hydrocarbon oil at 30°C is preferably 10 mPa·s or more, more preferably 30 mPa·s or more. From this viewpoint, the total content of isododecane, isohexadecane, and hydrogenated polyisobutene having viscosities of less than 10 mPa·s at 30°C is preferably 10 mass% or less, more preferably 5 mass% or less, further more preferably 1 mass% or less, even more preferably 0 .5 mass% or less, and may not be contained.

Similarly, from the standpoint of adhering the electrostatically sprayed coating to the skin, the viscosity of the ester oil and the silicone oil at 30°C is preferably 10 mPa·s or more, more preferably 30 mPa·s or more.

The viscosity here is measured at 30 °C by a type B viscometer (model TVB-10, manufactured by Toki Sangyo Co., Ltd.) and the measuring condition: Rotor No.1, 60 rpm, 1 minute. From the same viewpoint, the total content of the ether oils such as cetyl-1,3-dimethylbutyl ether, dicapryl ether, dilauryl ether, and diisostearyl ether in the liquid agent is preferably 10 mass% or less, more preferably 5 mass% or less, and even more preferably 1 mass% or less.

As the liquid oil, a polar oil in a liquid state at 20°C can also be preferably used, and examples thereof include ester oil, vegetable oil containing ester oil (triglyceride), higher alcohol of branched fatty acid or unsaturated fatty acid, preservative, silicone oil, and the like. These liquid oils can be used alone or in combination of two or more.

Examples of the ester oil include an ester consisting of a linear or branched chain fatty acid and a linear or branched chain alcohol or a polyvalent alcohol. Specifically, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethyleneglycol di(2-ethylhexanoate), dipentaerythritol fatty acid ester, N-alkylglycol monoisostearate, neopentylglycol dicaprate, diisostearyl malate, glyceryl di(2-heptylundecanoate), trimethylolpropane tri(2-ethylhexanoate), trimethylolpropane triisostearate, pentaerythritol tetra(2-ethylhexanoate), glyceryl tri(2-ethylhexanoate), trimethylolpropane triisostearate, cetyl 2-ethylhexanoate, 2-ethylhexyl palmitate, diethylhexyl naphthalenedicarboxylate, (C12-15)alkyl benzoate, cetearyl isononanoate, caprylic/capric triglyceride, dicaprylic/capric butyleneglycol, glyceryl trilaurate, glyceryl trimyristate, glyceryl tripalmitate, glyceryl triisostearate, glyceryl tri(2-heptylundecanoate), glyceryl tribehenate, glyceryl tricocoate, castor oil fatty acid methyl ester, oleyl oleate, 2-heptylundecyl palmitate, diisobutyl adipate, N-lauryol-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di(2-ethylhexyl) succinate, triethyl citrate, 2-ethylhexyl para-methoxycinnamate, tripropylene glycol dipivalate.

Among these, from the viewpoint of adhering the formed coating to the skin and in terms of excellent feeling of the coating upon application to the skin, at least one selected from the group consisting of octyldodecyl myristate, myristyl myristate, isocetyl stearate, isocetyl isostearate, cetearyl isononanoate, diisobutyl adipate, di-2-ethylhexyl sebacate, isopropyl myristate, isopropyl palmitate, diisostearyl malate, neopentylglycol dicaprate, (C12-15)alkyl benzoate, and caprylic/capric triglyceride, is preferable. At least one selected from the group consisting of isopropyl myristate, isopropyl palmitate, diisostearyl malate, neopentylglycol dicaprate, (C12-15)alkyl benzoate, and caprylic/capric triglyceride is more preferable. It is further preferable to contain one or more selected from the group consisting of neopentylglycol dicaprate, (C12-15)alkyl benzoate, and caprylic/capric triglyceride.

As the triglyceride, a fatty acid triglyceride is preferable, and examples of the triglyceride include, for example, an olive oil, a jojoba oil, a macadamia nut oil, a medform oil, a castor oil, a safflower oil, a sunflower oil, an avocado oil, a canola oil, an apricot kernel oil, a rice germ oil and a rice bran oil.

Examples of the higher alcohol include a liquid higher alcohol having 12 to 20 carbon atoms, specifically isostearyl alcohol, oleyl alcohol, and the like.

Examples of the preservative include phenoxyethanol, methyl para-hydroxybenzoate, ethyl para-aminobenzoate, isobutyl para-hydroxybenzoate, isopropyl para-hydroxybenzoate, ethyl para-hydroxybenzoate, butyl para-hydroxybenzoate, propyl para-hydroxybenzoate, benzyl para-hydroxybenzoate, and ethylhexanediol.

Examples of the silicone oil include dimethylpolysiloxane, dimethylcyclopolysiloxane, methylphenylpolysiloxane, methylhydrogenpolysiloxane, a higher alcohol modified organopolysiloxane, and the like. From the viewpoint of adhesivity to the skin, the content of the silicone oil in the liquid agent is preferably 10 mass% or less, more preferably 5 mass% or less, further more preferably 1 mass% or less, and even more preferably 0.1 mass% or less.

The kinematic viscosity of the silicone oil at 25°C is preferably 3 mm² per second, more preferably 4 mm² per second, even more preferably 5 mm² per second or more; preferably 30 mm² per second or less, more preferably 20 mm² per second or less, even more preferably 10 mm² per second or less, from the viewpoint of adhesivity of the electrostatically sprayed coating to the skin.

Among these, it is preferable that the silicone oil contain dimethylpolysiloxane from the viewpoint of adhesivity of the electrostatically sprayed coating to the skin.

The liquid agent preferably contains a liquid oil, and the content of the liquid oil in the liquid agent is preferably 0.1 mass% or more, more preferably 0.5 mass% or more, and even more preferably 5 mass% or more. It is preferably 100 mass% or less. The content of the liquid oil in the liquid agent is preferably 0.1 mass% or more and 100 mass% or less, more preferably 0.5 mass% or more and 100 mass% or less, and even more preferably 5 mass% or more and 100 mass% or less.

The liquid agent preferably contains a liquid oil or polyol, and the content of the liquid oil or polyol in the liquid agent is preferably 0.1 mass% or more, more preferably 0.5 mass% or more, and even more preferably 5 mass% or more. The content of the liquid oil or polyol in the liquid agent is preferably 100 mass% or less. The content of the liquid oil in the liquid agent is preferably 0. 1 mass% or more and 100 mass% or less, more preferably 0.5 mass% or more and 100 mass% or less, and even more preferably 5 mass% or more and 100 mass% or less.

When the liquid agent contains a polar oil, it is preferable that the liquid agent contain water and a polar oil from the viewpoint of enhancing adhesivity of the coating to the skin, and it is preferable that the liquid agent contain water and a polar oil in total of 40 mass% or more and 100 mass% or less. From the viewpoint of stability, the liquid agent may contain a surfactant, a polymer, a thickener, or an oil agent which is solid at 30°C, such as petrolatum, cetanol, stearyl alcohol, or ceramide, from the standpoint of improving adhesivity to the skin and moisturizing performance to the coating.

Similarly, when the liquid contains polyol, it is preferable that the liquid contain water and polyol from the viewpoint of enhancing adhesivity of the coating to the skin, and it is preferable that water and polyol contain in total of 40 mass% or more and 100 mass% or less. From the standpoint of stability, the liquid agent may contain a surfactant, a polymer, a thickener, or an oil agent which is solid at 30°C, such as petrolatum, cetanol, stearyl alcohol, ceramide, or the like, from the standpoint of improving adhesivity to the skin and moisturizing performance to the coating.

Whether a liquid agent uses water, polyol, or liquid oil, it is preferable that the liquid agent have a viscosity of about 5,000 mPa·s or less at 25°C from the viewpoint of improving adhesivity between the coating formed by the electrostatic spraying method and the skin. The method of measuring the viscosity of the liquid is as described above.

The content of the coloring pigment in the liquidagent is preferably less than 0.1 mass%, more preferably 0.05 mass% or less, further more preferably 0.01 mass% or less, even more preferably 0.001 mass% or less from the viewpoint of improving adhesivity between the coating formed by the electrostatic spraying method and the skin. In the present invention, the coloring pigment means that a transparent pigment is not contained, but a white pigment is contained.

Various methods can be used to apply liquid agents containing water or liquid oil to the skin by a method other than electrostatic spraying. For example, a thin layer of the liquid agent can be formed by applying the liquid agent to the skin by a method capable of applying the liquid to the skin such as dropping or sprinkling and, if necessary, by providing a step of spreading the liquid agent to conform to the skin or the coating. The step of spreading the solution may employ, for example, a method such as rubbing using a finger of the user or a tool such as an applicator. The liquid agent may be simply dropped or sprinkled, but providing the spreading step enables to conform the liquid agent to the skin or to the coating, which can sufficiently improve adhesivity of the coating. Alternatively, the liquid agent can be sprayed onto the skin to form a thin layer of the liquid agent. In this case, separate spreading is not particularly necessary, but the spreading operation after spraying is not hindered. When a liquid agent is applied after the formation of the coating, a sufficient amount of the liquid agent is applied to the skin, and the excess liquid can be removed by subjecting the liquid-applied area into contact with a sheet material, so that the excess liquid agent can be removed.

The amount of the liquid agent to be applied to the skin or to the coating may be an amount necessary and sufficient to improve the adhesivity between the skin and the coating. When a liquid oil is contained in the liquid agent, from the viewpoint of ensuring the adhesivity between the skin and the coating, the amount of the liquid agent to be applied to the skin is such that the basis weight of the liquid oil is preferably 0.1 g/m² or more, more preferably 0.2 g/m² or more; preferably 40 g/m² or less, more preferably 35 g/m² or less. The amount of the liquid agent to be applied to the skin is such that the basis weight of the liquid oil is preferably 0.1 g/m² or more and 40 g/m² or less, more preferably 0.2 g/m² or more and 35 g/m² or less.

In addition, the amount of the liquid agent to be applied to the skin or the coating is preferably 5 g/m² or more, more preferably 10 g/m² or more, more preferably 15 g/m² or more, preferably 50 g/m² or less, and more preferably 45 g/m² or less from the viewpoint of improving the adhesivity between the skin and the coating and improving the transparency.

Further, a cosmetic other than the liquid agent may be applied to the skin before or after the application of the liquid agent to the skin.

Applications using the present invention include a makeup cosmetic such as a makeup base, a foundation, a concealer, a blush, an eyeshadow, a mascara, an eyeliner, an eyebrow, an overcoat, a lipstick; a UV protective cosmetic such as a sunscreen cosmetic, a skin care cosmetics such as a moisturizing cosmetic, a wrinkle improving cosmetic, a whitening cosmetic, an anti-sebum cosmetic, an acne care cosmetic, and an aging care cosmetic. Among these, a makeup base, a foundation, a concealer, an overcoat, and a sunscreen cosmetic are more preferable.

Although the present invention has been described above with reference to its preferred embodiment, the present invention is not limited to the embodiments described above. For example, in the embodiment described above, a person who wants to form a coating on his or her own skin holds the electrostatic spraying device 10 and generates an electric field between the nozzle of the device 10 and his or her skin, but as long as an electric field is generated between the two, it is not necessary for him or her to hold the electrostatic spraying device 10.

With respect to the embodiments described above, the present invention further disclose methods for producing the following coatings.
<1> A method for producing a coating on skin, comprising a step of electrostatically spraying a composition comprising component (a), component (b) and component (c) directly onto the skin:
   (a) one or more volatile substances selected from the group consisting of water, an alcohol and a ketone,
   (b) a polymer having a film-forming ability,
   (c) a powder.
<2> The method according to <1>, wherein the step of electrostatically spraying forms a porous coating on the skin.
<3> The method according to <1> or <2>, wherein the electrostatic spraying is performed by using an electrostatic spraying device having a container containing the composition, a nozzle for discharging the composition therefrom, a supply device for supplying the composition contained in the container to the nozzle, and a power source for applying a voltage to the nozzle.
<4> The method according to any one of <1> to <3>, wherein the (a) volatile substance has a vapor pressure at 20°C of 0.01 kPa or more and 106.66 kPa or less, more preferably 0.13 kPa or more and 66.66 kPa or less, further more preferably 0.67 kPa or more and 40.00 kPa or less, and even more preferably 1.33 kPa or more and 40.00 kPa or less.
<5> The method according to any one of <1> to <4>, wherein the (a) volatile substance is an alcohol, and the alcohol is preferably one or more selected from the group consisting of a monovalent chain aliphatic alcohol, a monovalent cyclic aliphatic alcohol, and a monovalent aromatic alcohol, and more preferably one or more selected from the group consisting of ethanol, isopropyl alcohol, butyl alcohol, phenylethyl alcohol, propanol and pentanol.
<6> The method according to any one of <1> to <5>, wherein the (a) volatile substance is one or more selected from the group consisting of ethanol, isopropyl alcohol, butyl alcohol and water, preferably one or two selected from the group consisting of ethanol and butyl alcohol, more preferably one(s) comprising at least ethanol, and even more preferably one (s) comprising at least ethanol, and the content of ethanol in the volatile substance is 50 mass% or more and 100 mass% or less.
<7> The method according to any one <1> to <6>, wherein the (b) polymer having a film-forming ability is a substance that can be dissolved in the (a) volatile substance, and includes a water-soluble polymer and a water-insoluble polymer, (herein the term "dissolved" means a dispersed state at 20°C, and the dispersed state is visually uniform, preferably visually transparent or translucent).
<8> The method according to <7>, wherein the water-soluble polymer having a film-forming ability is one or more water-soluble polymer selected from the group consisting of mucopolysaccharide (e.g., pullulan, hyaluronic acid, chondroitin sulfate, poly-γ-glutamic acid, a modified corn starch, β-glucan, gluco-oligosaccharide, heparin, keratosulfuric acid), a natural polymer (e.g., cellulose, pectin, xylan, lignin, glucomannan, galacturonic acid, psyllium seed gum, tamarind seed gum, gum arabic, traganth gum, soy water-soluble polysaccharide, alginic acid, carrageenan, laminaran, agar (agarose), fucoidan, methyl cellulose, hydroxypropyl cellulose, and hydroxypropylmethyl cellulose, a partially saponified polyvinyl alcohol (not used in combination with a crosslinking agent), a low saponified polyvinyl alcohol, polyvinylpyrrolidone (PVP), polyethylene oxide and sodium polyacrylate; preferably one or more water-soluble polymers selected from the group consisting of pullulan, a partially saponified polyvinyl alcohol, a low saponified polyvinyl alcohol, polyvinyl pyrrolidone, and polyethylene oxide.
<9> The method according to <7>, wherein the polymer having a film-forming ability which is water-insoluble is one or more water-insoluble polymers selected from the group consisting of a fully saponified polyvinyl alcohol which can be insolubilized after the formation of a coating, a partially saponified polyvinyl alcohol which can be crosslinked by using in combination with a crosslinking agent, an oxazoline-modified silicone such as poly(N-propanoylethyleneimine)graft-dimethylsiloxane/γ-aminopropyl methylsiloxane copolymer, polyvinyl acetate diethylaminoacetate, Zein (a main component of corn protein), a polyester, polylactic acid (PLA), an acrylic resin (e.g., a polyacrylonitrile resin, a polymethacrylic acid resin), a polystyrene resin, a polyvinyl butyral resin, a polyethylene terephthalate resin, a polybutylene terephthalate resin, a polyurethane resin, a polyamide resin, a polyimide resin, and a polyamide-imide resin; more preferably one or more water-insoluble polymers selected from the group consisting of a fully saponified polyvinyl alcohol which can be insolubilized after the formation of a coating, a partially saponified polyvinyl alcohol which can be cross linked by using in combination with a cross linking agent, a polyvinyl butyral resin, an oxazoline-modified silicone (e.g., poly(N-propanoylethyleneimine)graft-dimethylsiloxane/γ-aminopropyl methylsiloxane copolymer), a water-soluble polyester and Zein.
<10> The method according to any one of <1> to <9>, wherein component (b) is preferably one or more selected from the group consisting of a fully saponified or partially saponified polyvinyl alcohol, a polyvinyl butyral resin, a polyurethane resin, an oxazoline-modified silicone, a water-soluble polyester, and Zein, and more preferably one or more selected from the group consisting of a polyvinyl butyral resin and a polyurethane resin.
<11> The method according to any one of <1> to <10>, wherein the content of component (a) in the composition is preferably 30 mass% or more, more preferably 55 mass% or more, further more preferably 60 mass% or more; preferably 98 mass% or less, more preferably 96 mass% or less, further more preferably 94 mass% or less, and the content of component (a) in the composition is preferably 30 mass% or more and 98 mass% or less, more preferably 55% or more and 96 mass% or less, and further more preferably 60 mass% or more and 94 mass% or less.
<12> The method according to any one of <1> to <11>, wherein the content of component (b) in the composition is preferably 2 mass% or more, more preferably 4 mass% or more, further more preferably 6 mass% or more; preferably 50 mass% or less, more preferably 45 mass% or less, further more preferably 40 mass% or less; preferably 2 mass% or more and 50 mass% or less, more preferably 4% or more and 45 mass% or less, and even more preferably 6 mass% or more and 40 mass% or less.
<13> The method according to any one of <1> to 12 >, wherein the content ratio of component (a) to component (b), ((a)/(b)), is 0.5 or more and 40 or less, preferably 1 or more and 30 or less, and more preferably 2 or more and 25 or less.
<14> The method according to any one of <1> to <12>, wherein the content ratio of content (a) (ethanol) to component (b), ((a)/(b)), is 0.5 or more and40 or less, preferably 1 or more and 30 or less, and more preferably 2 or more and 25 or less.
<15> The method according to any one of <1> to <14>, wherein component (c) is one or more selected from the group consisting of a coloring pigment, a constitutive pigment, a pearl pigment and an organic powder, preferably one or more selected from the group consisting of an inorganic coloring pigment, an organic coloring pigment, an organic dye, a constitutive pigment, a pearl pigment and an organic powder.
<16> The method according to <15>, wherein the inorganic coloring pigment is one or more selected from the group consisting of an inorganic coloring pigment such as red iron oxide, iron hydroxide, iron titanate, yellow iron oxide, black iron oxide, carbon black, dark blue, ultramarine blue, dark blue titanium oxide, black titanium oxide, sintered titanium/titanium oxide, manganese violet, cobalt violet, chromium oxide, chromium hydroxide, cobalt oxide, cobalt titanate; and inorganic white pigment such as titanium oxide, zinc oxide, calamine, zirconium oxide, magnesium oxide, cerium oxide, aluminum oxide, and composites thereof, preferably one or more selected from the group consisting of iron oxide, titanium oxide and zinc oxide, and more preferably one or more selected from the group consisting of titanium oxide, zinc oxide, red iron oxide, yellow iron oxide and black iron oxide.
<17> The method according to <15>, wherein the organic coloring pigment/organic dye is an organic tar pigment such as red No. 3, red No. 102, red No. 104, red No. 106, red No. 201, red No. 202, red No. 204, red No. 205, red No. 220, red No. 226, red No. 227, red No. 228, red No. 230, red No. 401, red No. 405, red No. 505, orange No. 203, orange No. 204, orange No. 205, yellow No. 4, yellow No. 5, yellow No. 401, blue No. 1 and blue No. 404; and an organic dye such as β-carotene, caramel and paprika.
<18> The method according to <18>, wherein the constitutive pigment is one or more selected from the group consisting of barium sulfate, calcium sulfate, magnesium sulfate, magnesium carbonate, calcium carbonate, talc, mica, kaolin, sericite, silicic acid, anhydrous silicic acid, aluminum silicate, magnesium silicate, magnesium aluminum silicate, calcium silicate, barium silicate, strontium silicate, metal tungstate, hydroxyapatite, vermiculite, clay, bentonite, montmorillonite, hectorite, smectite, zeolite, ceramic powder, dibasic calcium phosphate, alumina, silica, aluminum hydroxide, boron nitride, syntheticmica, synthetic sericite, metal soap, barium sulfate and barium sulfate-treated mica; preferably one or more selected from the group consisting of barium sulfate, calcium carbonate, mica, anhydrous silicic acid, talc, boron nitride and synthetic mica.
<19> The method according to <15>, wherein the pearl pigment (glittering powders) is selected from the group consisting of fish foil, titanium oxide-coated mica (mica titanium), bismuth oxychloride, titanium oxide-coated bismuth oxychloride, titanium oxide-coated talc, titanium oxide-coated color mica, (titanium oxide/iron oxide)-coated mica, microparticulate titanium oxide-coated mica titanium, microparticulate zinc oxide-coated mica titanium, organic pigment-treated mica titanium, low-order titanium oxide-coated mica, titanium oxide-coated synthetic mica, titanium oxide-coated platy silica, hollow plate-like titanium oxide, iron oxide-coated mica, platy iron oxide (MIO), an aluminum flake, a stainless flake, titanium oxide-coated plate alumina, a glass flake, a titanium oxide-coated glass flake, pearl shell, gold foil, a gold vapor-coated resin film, and a metal vapor-coated resin film. One or more thereof can be used.
<20> The method according to <15>, wherein the organic powder is one or more selected from the group consisting of a silicone rubber powder, a silicone resin-coated silicone rubber powder, a polymethylsilsesquioxane, a polyamide powder, a nylon powder, a polyester powder, a polypropylene powder, a polystyrene powder, a polyurethane powder, a vinyl resin powder, a urea resin powder, a phenolic resin powder, a fluorine resin powder, a silicon resin powder, an acrylic resin powder, a melamine resin powder, a polycarbonate resin, a divinylbenzene-styrene copolymer, a silk powder, a wool powder, a cellulose powder, a long-chain alkyl phosphoric acid metal salt, N-mono long-chain alkylacyl basic amino acid and a composite thereof; preferably one or more selected from the group consisting of a cellulose powder, a silicone rubber powder, a silicone resin-coated silicone rubber powder, polymethylsilsesquioxane, an acrylic resin powder and a nylon powder.
<21> The method according to any one of <15> to <20>, wherein the powder is a powder that has been subjected to hydrophobic treatment, and is preferably a powder that has been subjected to hydrophobic treatment with one selected from the group consisting of a fluorine compound, a silicone compound, a metal soap, an amino acid compound, lecithin, an alkylsilane, an oil agent, and an organic titanate.
<22> The method according to any one of <15> to <20>, wherein the powder is a powder that has been subjected to hydrophilic treatment.
<23> The method according to any one of <1> to <22>, wherein average particle diameter of the powder is preferably 0.001 µm or more and 200 µm or less, more preferably 0.01 µm or more and 50 µm or less, further more preferably 0.02 µm or more and 20 µm or less, and even more preferably 0.05 µm or more and 10 µm or less.
<24> The method according to any one of <1> to <23>, wherein the content of component (c) in the composition is 0.001 mass% or more, preferably 0.01 mass% or more, more preferably 0.1 mass% or more, and is 50 mass% or less, preferably 30 mass% or less, and more preferably 15 mass% or less.
<25> The method according to any one of <1> to <24>, wherein the content ratio of component (c) to component (b) in the composition, ((c)/(b)), is preferably 0.001 or more and 20 or less, more preferably 0.002 or more and 1 or less, and even more preferably 0.005 or more and 0.5 or less.
<26> The method according to any one of <1> to <25>, wherein the mass ratio of the coloring pigment to the total powder (component (c)) in the composition, (coloring pigment/(c)), is preferably 0.2 or more, more preferably 0.3 or more, even more preferably 0.4 or more; preferably 1.0 or less.
<27> The method according to any one of <1> to <26>, wherein the composition further comprises an oil agent.
<28> The method according to <27>, wherein the oil agent is preferably a polar oil in a liquid state at 20°C, and is one or more selected from the group consisting of a hydrocarbon oil, an ester oil, an ether oil, a higher alcohol, and a silicone oil.
<29> The method according to <27> or <28>, wherein the content of the oil agent is preferably 0.01 mass% or more, more preferably 0.1 mass% or more; preferably 30 mass% or less, more preferably 20 mass% or less, and even more preferably 10 mass% or less.
<30> The method according to anyone of <1> to <29>, wherein the viscosity of the composition at 25°C is preferably 1 mPa·s or more, more preferably 10 mPa·s or more, even more preferably 50 mPa·s or more; preferably 5,000 mPa·s or less, more preferably 2,000 mPa·s or less; preferably 1 mPa·s or more and 5,000 mPa·s or less, more preferably 10 mPa·s or more and 2,000 mPa·s or less, and even more preferably 50 mPa·s or more and 1,500 mPa·s or less.
<31> The method according to any one of <1> to <30>, wherein the composition further comprises another component selected from the group consisting of a plasticizer of (b) polymer having a film-forming ability, a surfactant, a UV protection agent, a perfume, a repellent, an antioxidant, a stabilizer, a preservative and various vitamins.
<32> The method according to <31>, wherein when the other component (s) is (are) included in the composition, the content ratio thereof is preferably 0.1 mass% or more and 30 mass% or less, and more preferably 0.5 mass% or more and 20 mass% or less.
<33> The method according to any one of <1> to <32>, wherein the electrostatic spraying is performed by using an electrostatic spraying device, the electrostatic spraying device comprises a nozzle, the nozzle is made of a conductive material such as metal or a non-conductive material such as plastic, rubber, or ceramic, and the nozzle has a shape capable of discharging the composition from the tip thereof.
<34> The method according to any one of <1> to <33>, wherein the electrostatic spraying is performed by using an electrostatic spraying device, the electrostatic spraying device comprises a nozzle and a housing, the nozzle is disposed at one longitudinal end of the housing, and the nozzle is disposed in the housing such that a blowing direction of the composition coincides with the longitudinal direction of the housing and is convex toward the skin side.
<35> The method according to any one of <1> to <34>, which forms a fiber by stretching and deforming the sprayed composition by a potential difference, while volatilizing a volatile substance (a solvent), from a droplet to solidify a polymer (a solute having a film-forming ability) .
<36> The method for producing a coating according to any of <1>to <35>, wherein the electrostatic spraying is performed by using an electrostatic spraying device, the electrostatic spraying device comprises a nozzle, and the distance between the nozzle and the skin is 50 mm or more and 150 mm or less.
<37> The method according to any one of <1> to <36>, wherein the basis weight of the coating formed by the electrostatic spraying method is preferably 0.1 g/m² or more, more preferably 1 g/m² or more; preferably 30 g/m² or less, more preferably less than 20 g/m² or less; preferably 0.1 g/m² or more and 30 g/m² or less, and more preferably 1 g/m² or more and 20 g/m² or less.
<38> The method according to any one of <1> to <37>, comprising a step of applying a liquid agent comprising one or more selected from the group consisting of water, polyol, and an oil in a liquid state at 20°C to the skin by a method other than an electrostatic spraying before and/or after the electrostatic spraying step of forming the coating by the electrostatic spraying.
<39> The method according to <38>, wherein when the liquid agent comprises water, the liquid agent includes a water-based liquid such as water, an aqueous solution and an aqueous dispersion, and the liquid agent may include a cosmetic water; an emulsion composed of an emulsified liquid such as an O/W emulsion or a W/O emulsion; an aqueous solution thickened with a thickener.
<40> The method according to <38> or <39>, wherein when the liquid agent used in the liquid agent application step includes an oil in a liquid state at 20 °C, the liquid oil may include a linear or branched hydrocarbon oil such as liquid paraffin, light isoparaffin, liquid isoparaffin, squalane, and squalene; a vegetable oil such as a jojoba oil, and an olive oil; an animal oil such as a liquid lanolin; an ester oil such as a monoalcohol fatty acid ester, and a polyvalent alcohol fatty acid ester; a silicone oil such as dimethylpolysiloxane, dimethylcyclopolysiloxane, methylphenylpolysiloxane, methylhydrogenpolysiloxane, and a high alcohol modified organopolysiloxane; preferably a hydrocarbon oil, an ester oil, a vegetable oil containing a triglyceride, and apolar oil such as a silicone oil; more preferably one or more selected from the group consisting of a hydrocarbon oil, an ester oil and a triglyceride.
<41> The method according to anyone of <38> to <40>, wherein the liquid agent preferably comprises a liquid oil, and the content of the liquid oil in the liquid agent is preferably 0.1 mass% or more, more preferably 0.5 mass% or more, even more preferably 5 mass% or more; preferably 100 mass% or less; preferably 0.1 mass% or more and 100 mass% or less, more preferably 0.5 mass% or more and 100 mass% or less, even more preferably 5 mass% or more and 100 mass% or less.
<42> The method according to any one of <38> to <41>, wherein when the liquid agent comprises a polar oil, the liquid agent preferably comprises water and a polar oil, more preferably the liquid agent comprises water and polar oil in total of 40 mass% or more and 100 mass% or less.
<43> The method according to any one of <38> to <42>, wherein the liquid agent preferably comprises a surfactant, a polymer, and/or a thickener, preferably an oil in a solid state at 30°c such as vaseline, cetanol, stearyl alcohol, or ceramide.
<44> The method according to any one of <38> to <43>, wherein when the liquid agent comprises a liquid oil, the amount of the liquid agent to be applied to the skin is such that the basis weight of the liquid oil is preferably 0.1 g/m² or more, more preferably 0.2 g/m² or more; preferably 40 g/m² or less, more preferably 35 g/m² or less; preferably 0.1 g/m² or more and 40 g/m² or less, more preferably 0.2 g/m² or more and 35 g/m² or less, and the amount of the liquid agent to be applied to the skin or to the coating is preferably 5 g/m², more preferably 10 g/m² or more, more preferably 15 g/m² or more; preferably 50 g/m² or less, and more preferably 45 g/m² or less.
<45> The method according to any one of <1> to <44>, wherein the composition further comprises 10 mass% or less of a glycol.
<46> The method according to any one of <1> to <45>, wherein the composition is used as a cosmetic selected from the group consisting of a makeup cosmetic, a UV protective cosmetic, and a skincare cosmetic .
<47> The method according to any one of <1> to <46>, wherein component (a) is a volatile substance comprising at least ethanol, and component (b) is one or more selected from the group consisting of a polyvinyl butyral resin and a polyurethane resin.
<48> The method according to any one of <1> to <47>, wherein component (a) is avolatile substance comprising at least ethanol and the composition further comprises 10 mass% or less of a glycol.
<49> The method according to any one of <1> to <48>, wherein component (a) is a volatile substance comprising at least ethanol, the content of the ethanol in the volatile substance is 50 mass% or more and 100 mass% or less, and the composition comprises 10 mass% or less of a glycol.
<50> The method according to any one of <1> to <49>, wherein the content of component (a) in the composition is 30 mass% or more and 98 mass% or less, the content of component (b) in the spraying composition is 2 mass% or more and 50 mass% or less, and the content of component (c) in the spraying composition is 0.001 mass% or more and 50 mass% or less.
<51> The method according to any one of <1> to <49>, wherein the content of component (a) in the composition is 55 mass% or more and 96 mass% or less, the content of component (b) in the spraying composition is 4 mass% or more and 45 mass% or less, and the content of component (c) in the spraying composition is 0.01 mass% or more and 30 mass% or less.
<52> The method according to any one of <1> to <49>, wherein the content of component (a) in the composition is 60 mass% or more and 94 mass% or less, the content of component (b) in the spraying composition is 6 mass% or more and 40 mass% or less, and the content of component (c) in the spraying composition is 0.1 mass% or more and 15 mass% or less.
<53> The method according to any one of <1> to <52>, wherein component (a) is a volatile substance comprising at least ethanol, and component (b) is one or more selected from the group consisting of a polyvinyl butyral resin and a polyurethane resin.
<54> The method according to any one of <1> to <53>, wherein component (a) is a volatile substance comprising at least ethanol and the composition further comprises 10 mass% or less of a glycol.
<55> The method according to any one of <1> to <54>, wherein component (a) is a volatile substance comprising at least ethanol, the content of the ethanol in the volatile substance is 50 mass% or more and 100 mass%, and the content of a glycol is 10 mass% or less.

### [Examples]

The present invention will be explained in more detail by means of the following examples. However, the scope of the present invention is not limited to these examples . Unless otherwise specified, "%" means "mass %".

### [Test Example 1]

### [Examples 1 to 33]

### (1) Preparation of spraying compositions

The compositions described in Tables 1 to 8 were prepared.

### (2) Electrostatic spraying process

An electrostatic spraying device 10 having the configuration shown in FIG. 1 and having the appearance shown in FIG. 2 was used, and an electrostatic spraying method was performed toward a cosmetic application site for 20 seconds. The conditions of the electrostatic spraying method were as follows.
- Applied voltage: 10 kV
- Distance between conductive nozzle and skin: 100 mm
- Volume of spraying composition: 5 mL/h
- Environment :25°C, 30%RH

The electrostatic spray formed a porous coating of a deposit of fibers across the cosmetic application site. The coating was a circle approximately 4 centimeters in diameter and the mass was approximately 5.5 mg. The fiber thickness measured by the method described above was 1 µm.

### [Comparative Examples 1 to 3]

The compositions described in Table 9 were prepared.
These compositions were applied in an amount of 0.2 g/400 cm² to the skin. No electrostatic spraying was performed.

### Evaluation

Examples and comparative examples shown in Tables 1 to 9 were produced and evaluated for "aggregation of the powder on the skin immediately after application", "stickability to skin hills, skin grooves and skin pores", "no uneven color of the finished skin", and "no white float on finished skin". The results are given in Tables 1 to 9.

### (1) Powder aggregation on the skin immediately after application

The expert panelist evaluated " immediately after application, the aggregation of the powder on the skin" when actually spraying the spraying composition (foundation) onto the skin by the following criteria. Results are presented as the average of five panelists.

### Evaluation criteria

4: No powder agglomeration is perceived on the skin.
3: Slight powder agglomerations are perceived on the skin.
2: Powder agglomerations are perceived on the skin.
1: Many powder agglomerations are perceived on the skin.

### (2) Stickability to skin hills, skin grooves and skin pores

The expert panelists evaluated "stickability to skin hills, skin grooves and skin pores" when they actually sprayed the spraying composition (foundation) onto the skin according to the following criteria. Results are presented as the average of five panelists.

### Evaluation criteria

4: The powder sticks uniformly to the whole skin.
3: The powder sticks slightly unevenly to the whole skin.
2: The powder penetrates into the skin groove and pore of the skin and sticks unevenly.
1: The powder does not stick to the skin hills, but enters the skin grooves and skin pores, and sticks fairly unevenly.

### (3) Non-uniform color of finished skin

The expert panelists evaluated the "non-uniformity of the finished skin color" when they actually sprayed the spraying composition (foundation) onto the skin according to the following criteria. Results are presented as the average of five panelists.

### Evaluation criteria

4: No unevenness is perceived on the skin after application.
3: Not so much unevenness is perceived on the skin after application.
2: Slight unevenness is perceived on the skin after application.
1: Unevenness is observed on the skin after application.

### (4) No white float on finished skin

The expert panelists evaluated the "no white float on finished skin" when they actually sprayed the spraying composition (foundation) onto the skin according to the following criteria. Results are presented as the average of five panelists.

### • Evaluation criteria

4: No white float was perceived on the skin after application.
3: Not so much white float is perceived on the skin after application.
2: Slight white float is perceived on the skin after application.
1: White float is perceived on the skin after application.

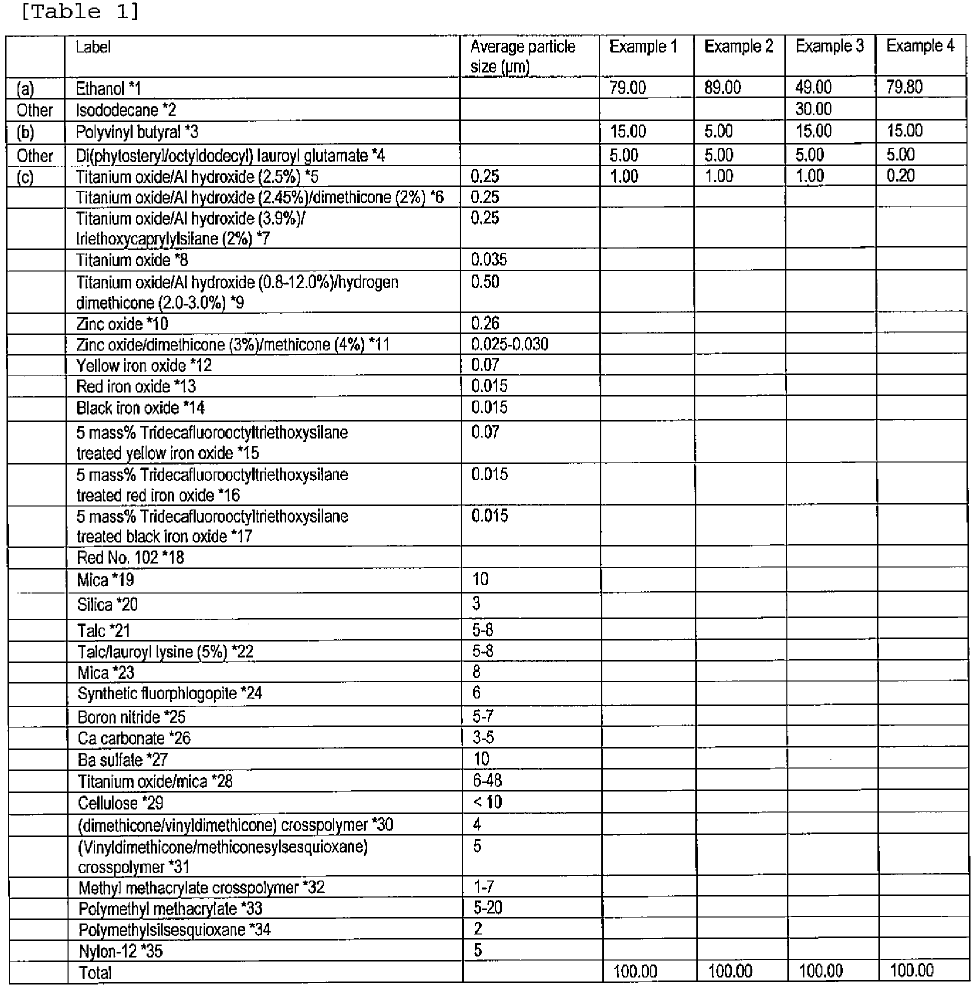

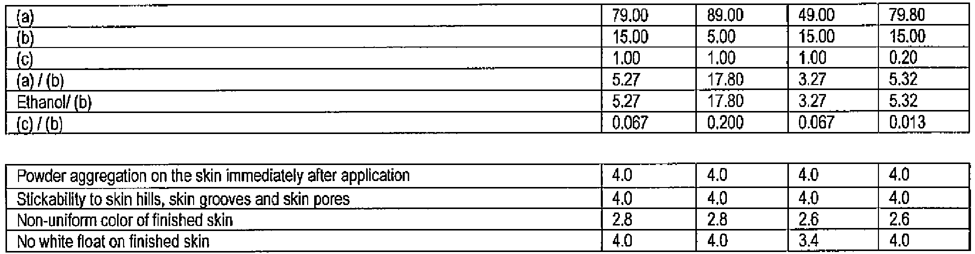

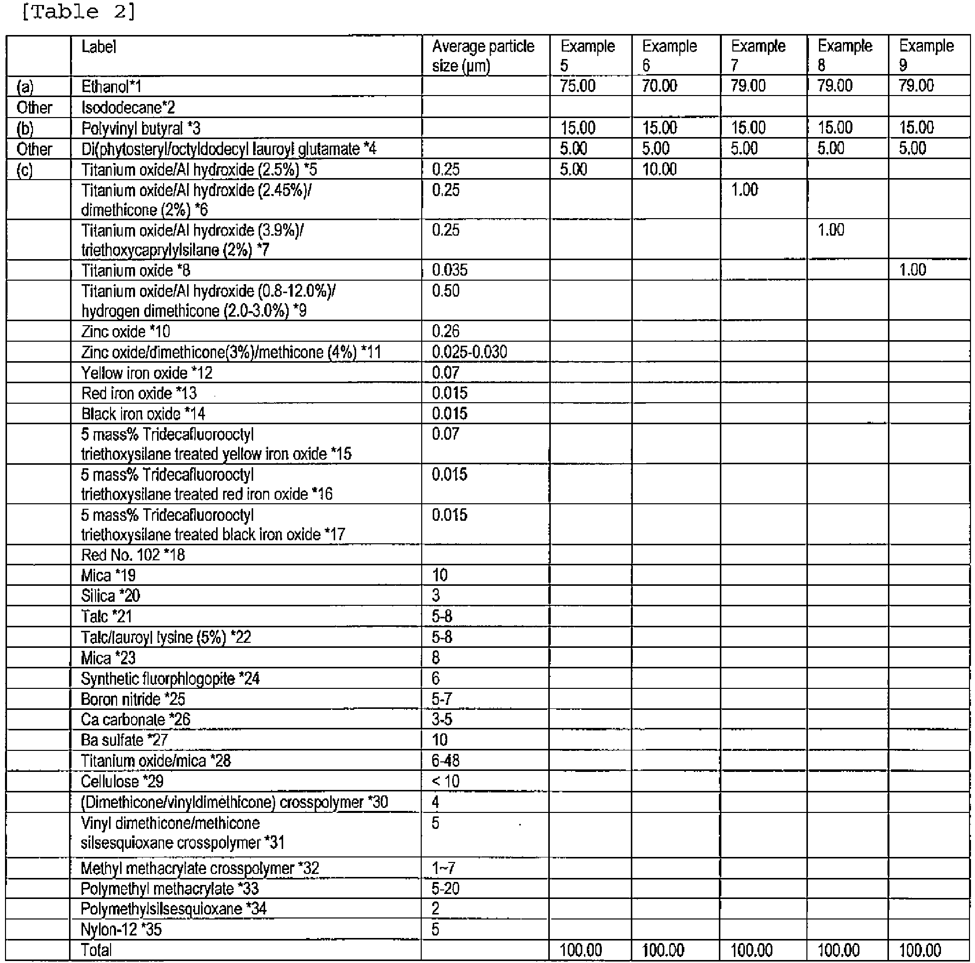

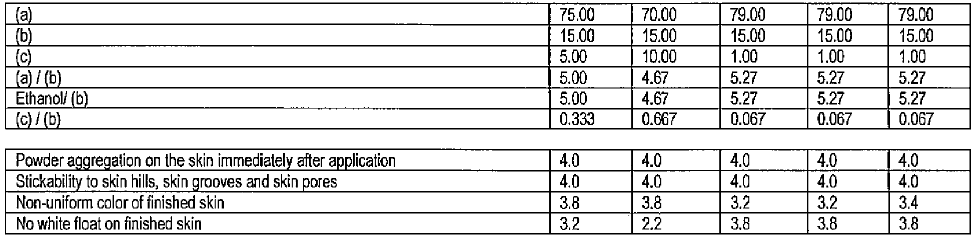

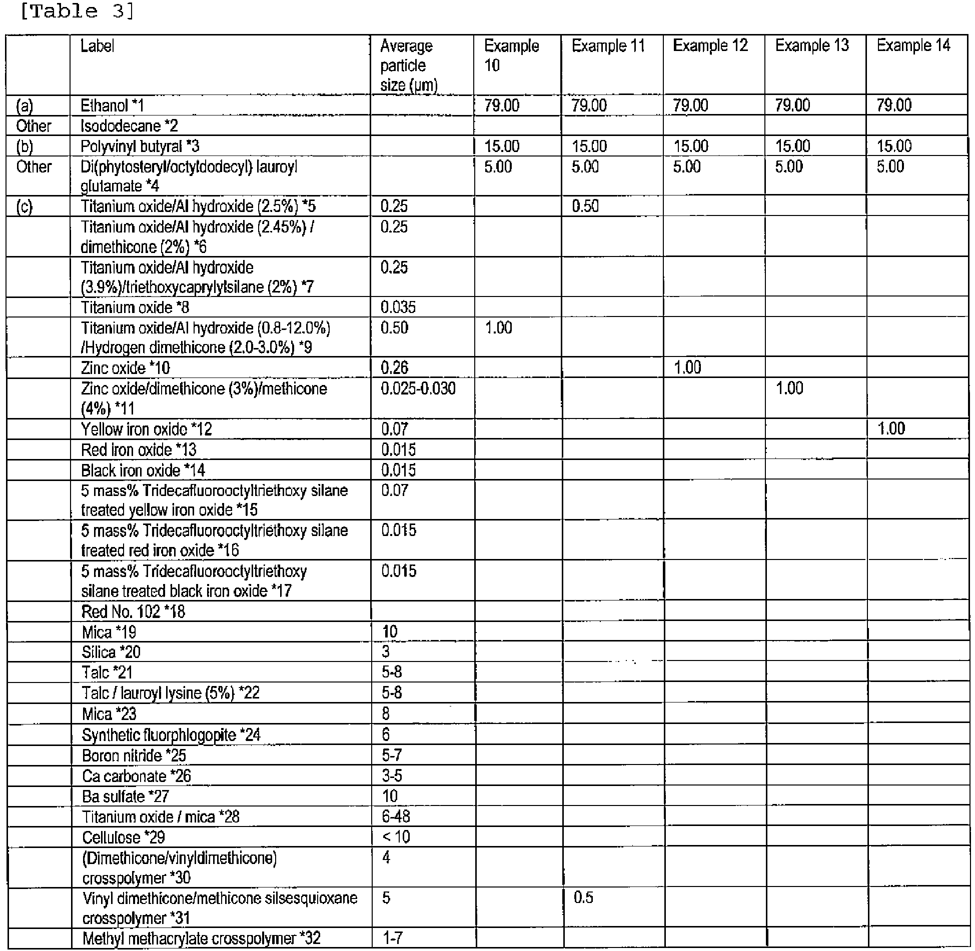

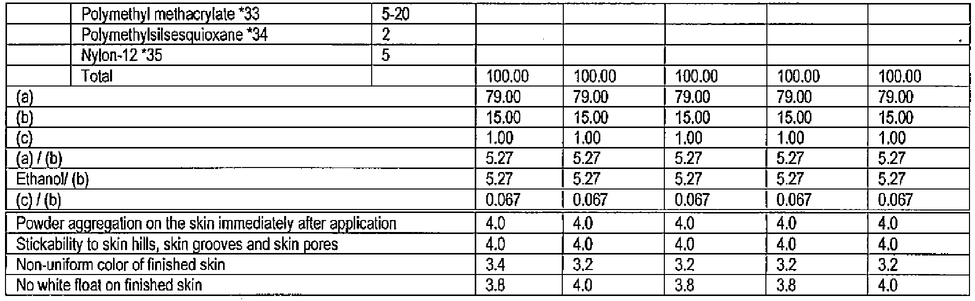

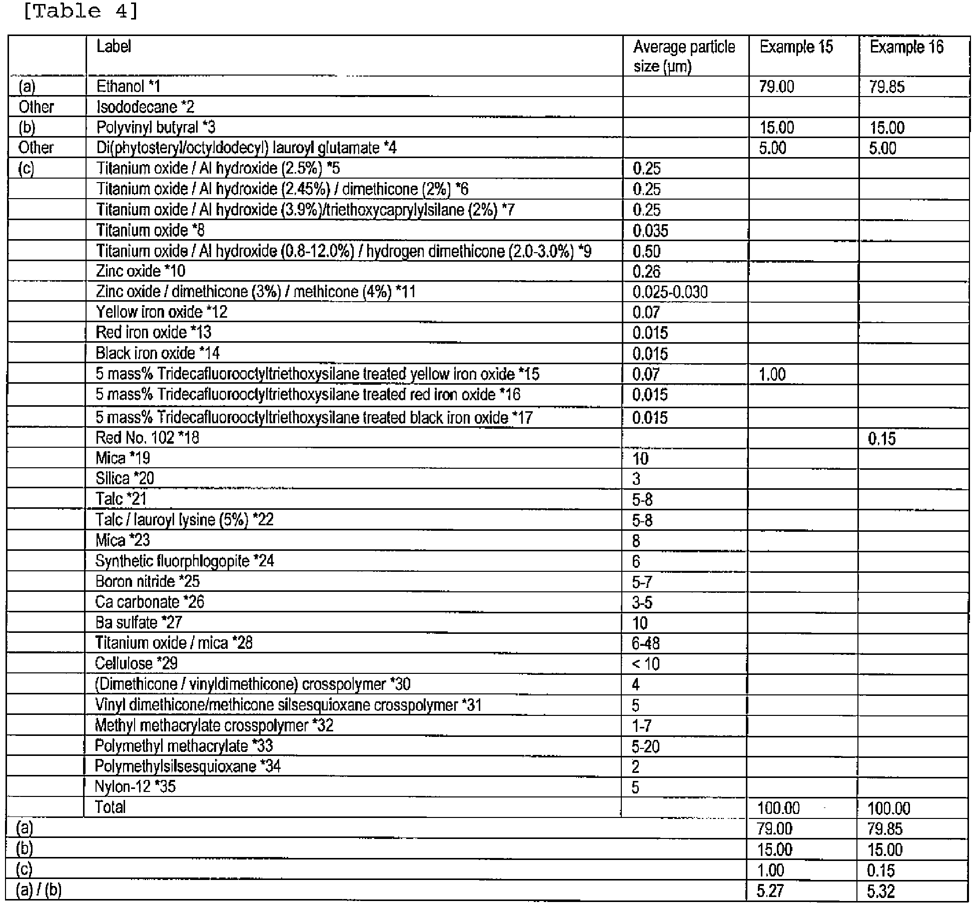

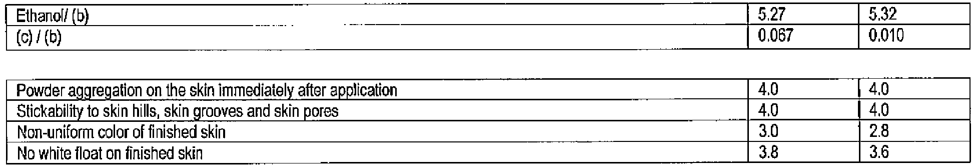

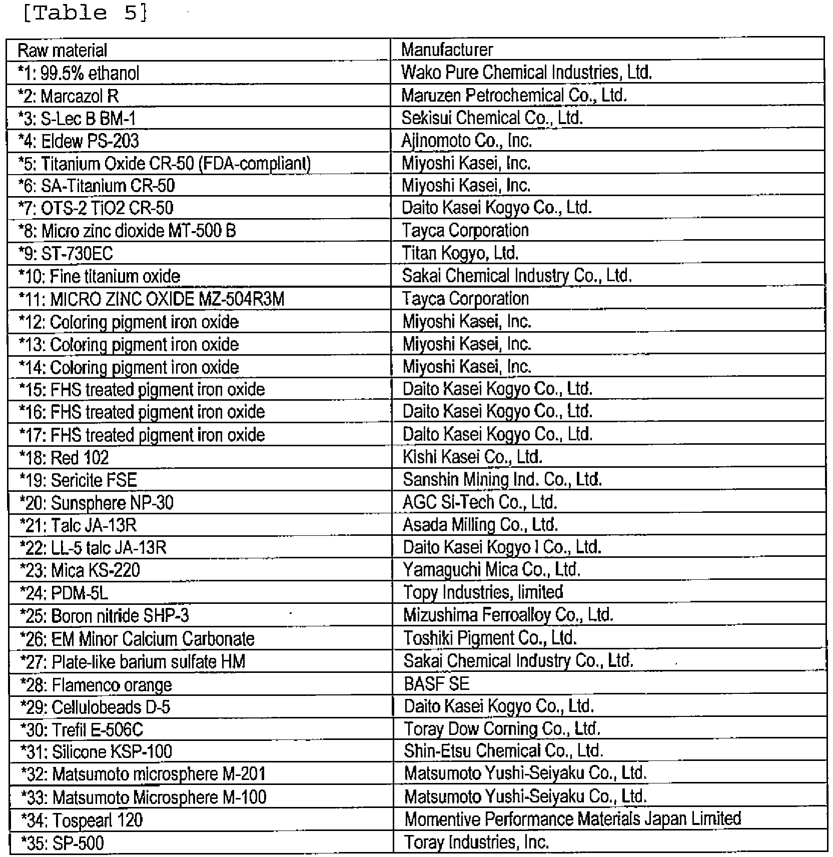

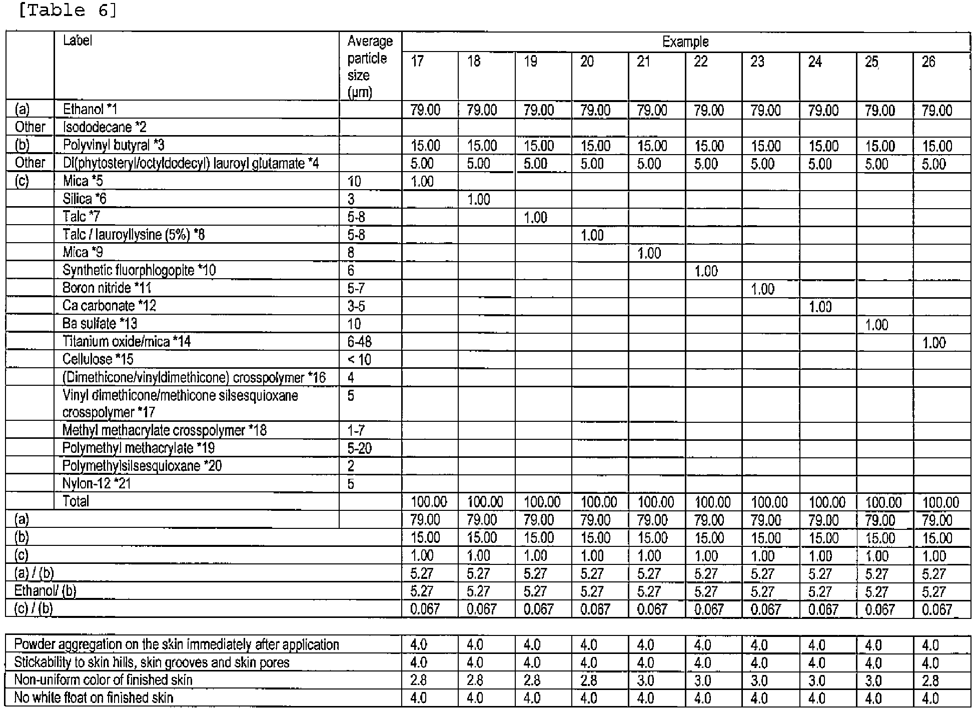

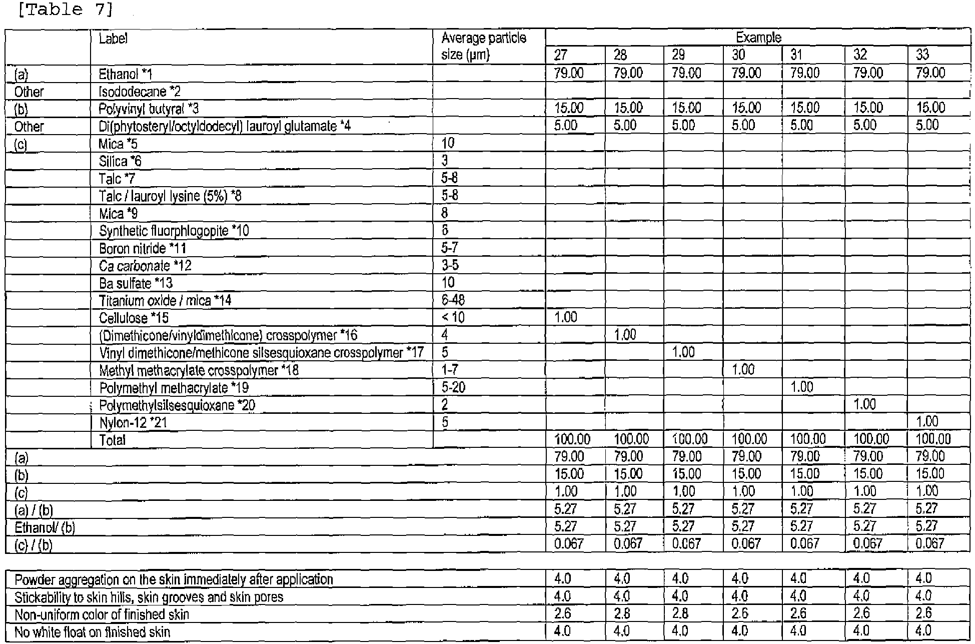

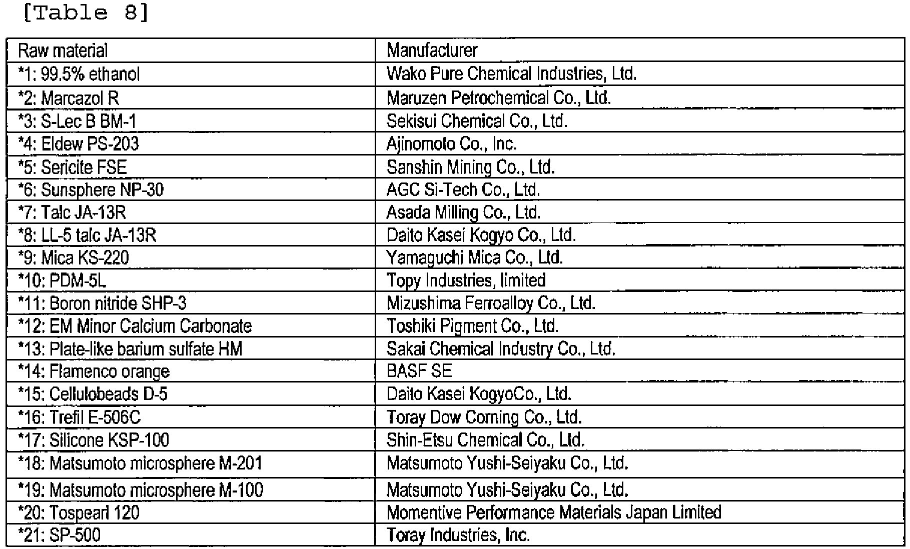

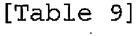

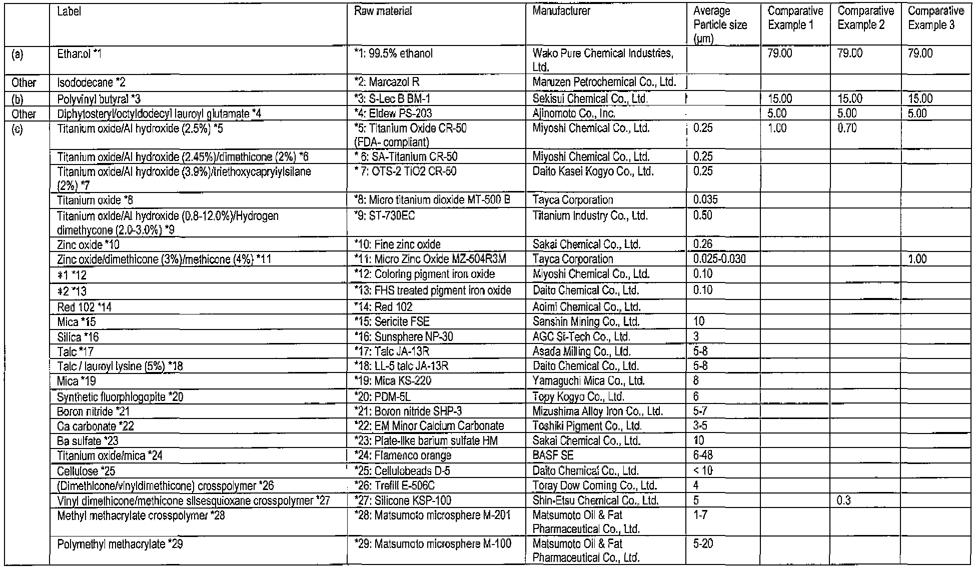

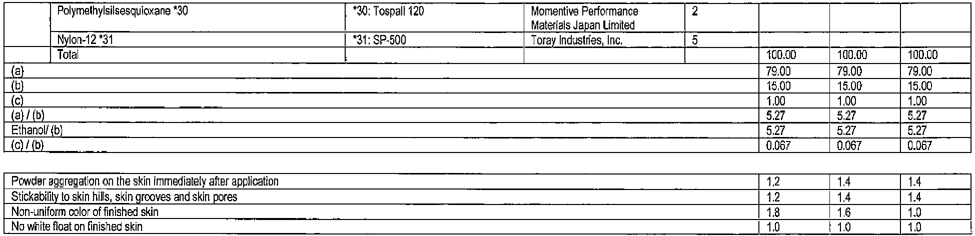

### Examples 34 and 35

The compositions described in Table 10 were prepared. The composition can be electrostatically sprayed onto the skin as a spraying composition.

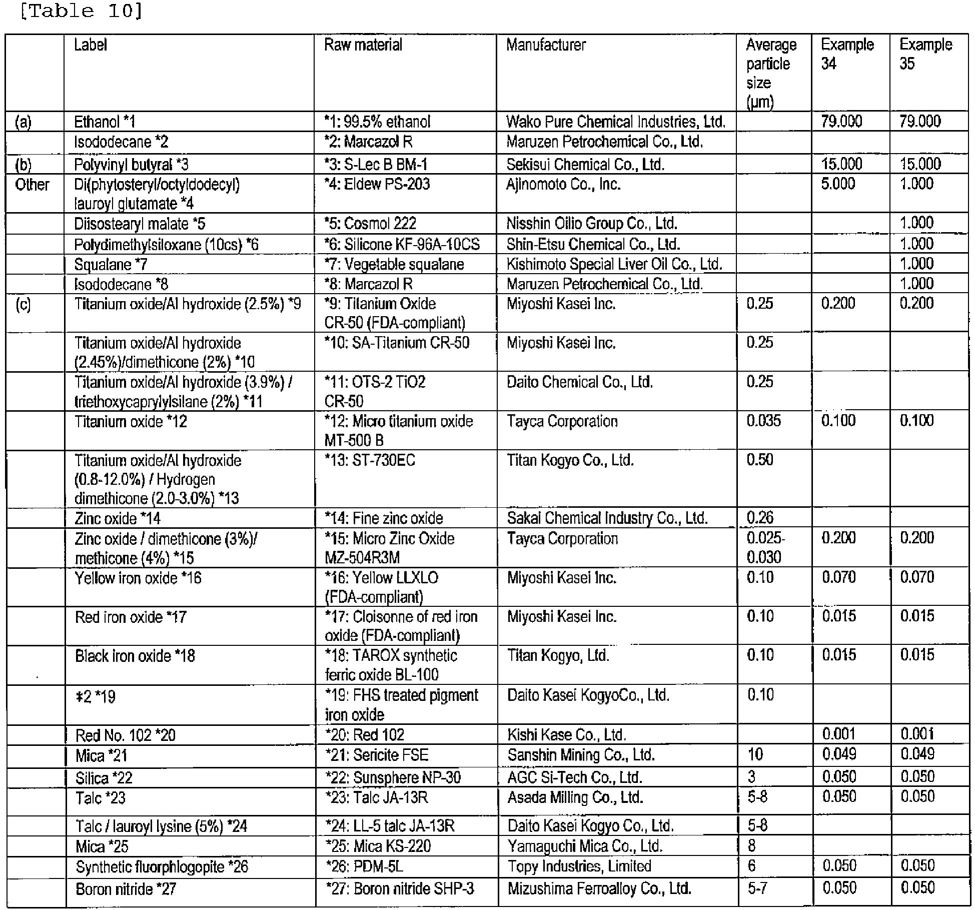

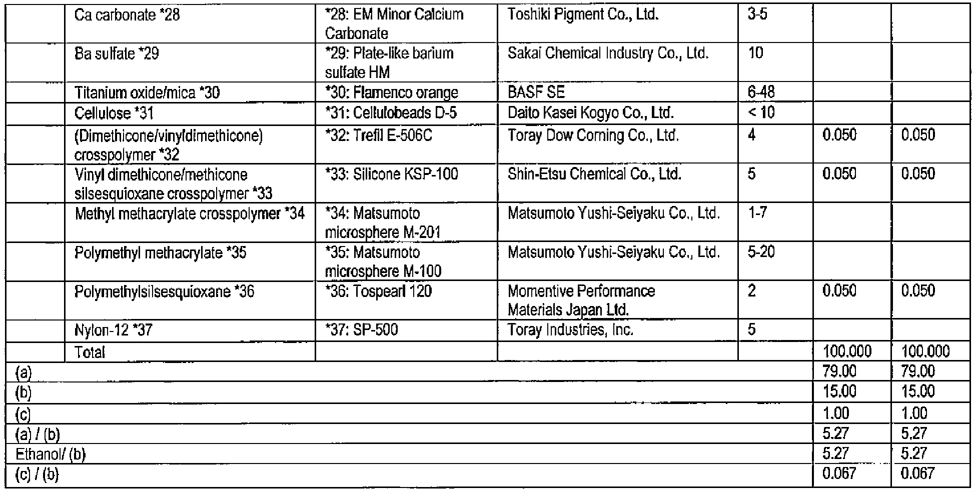

### [Examples 36 to 47]

Compositions of Tables 11 to 15 were prepared. This composition was electrostatically sprayed as a spraying composition in the same manner as in Examples 1 to 34. The results are also shown in Tables 11 to 15.

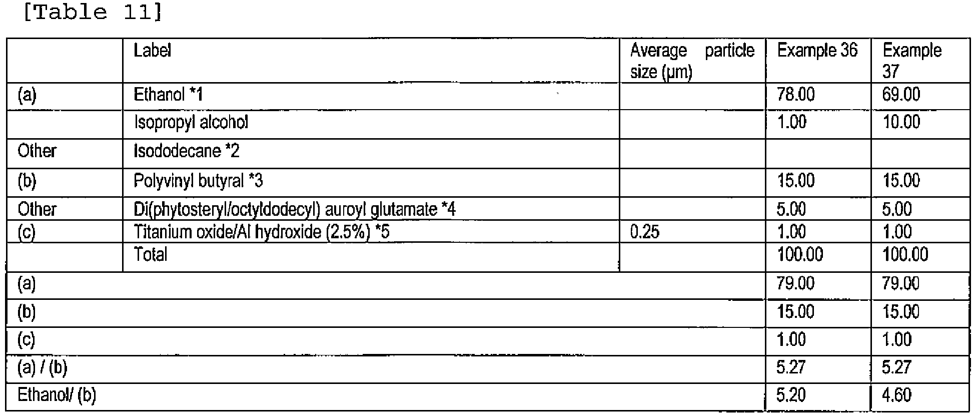

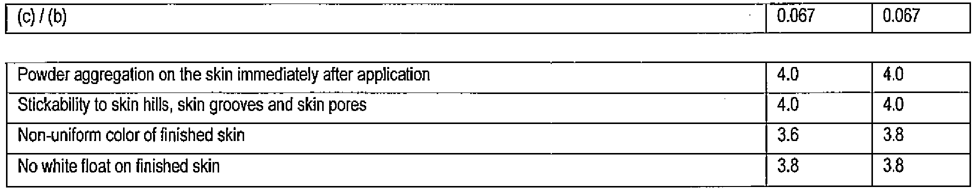

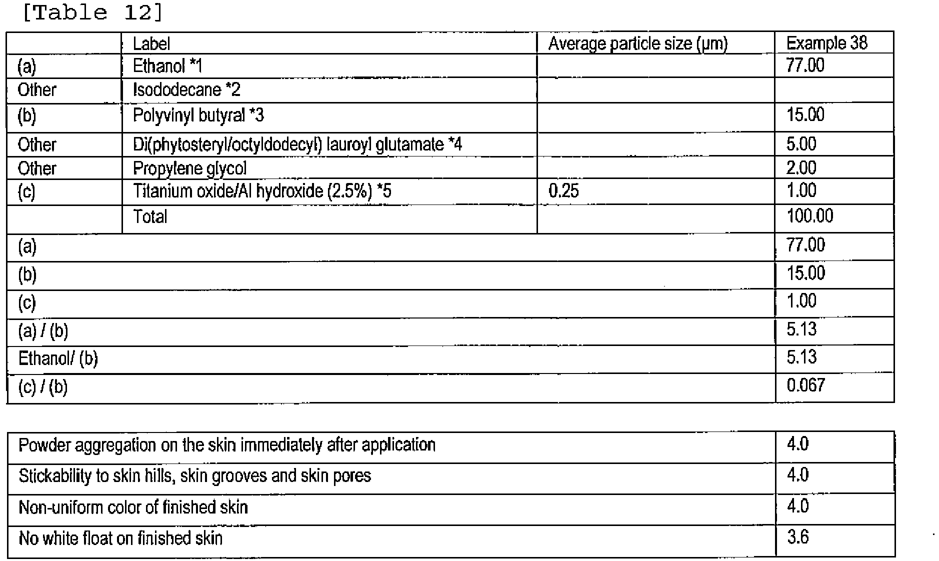

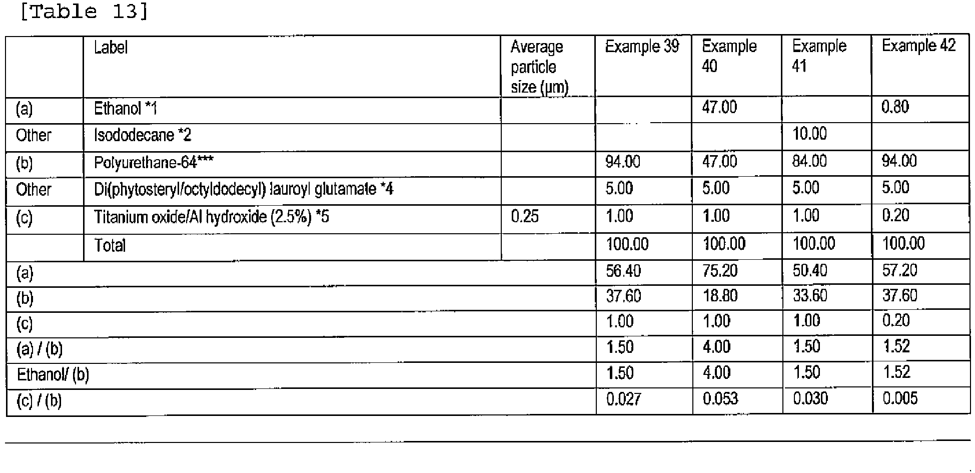

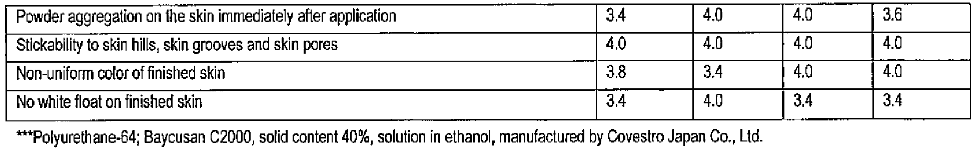

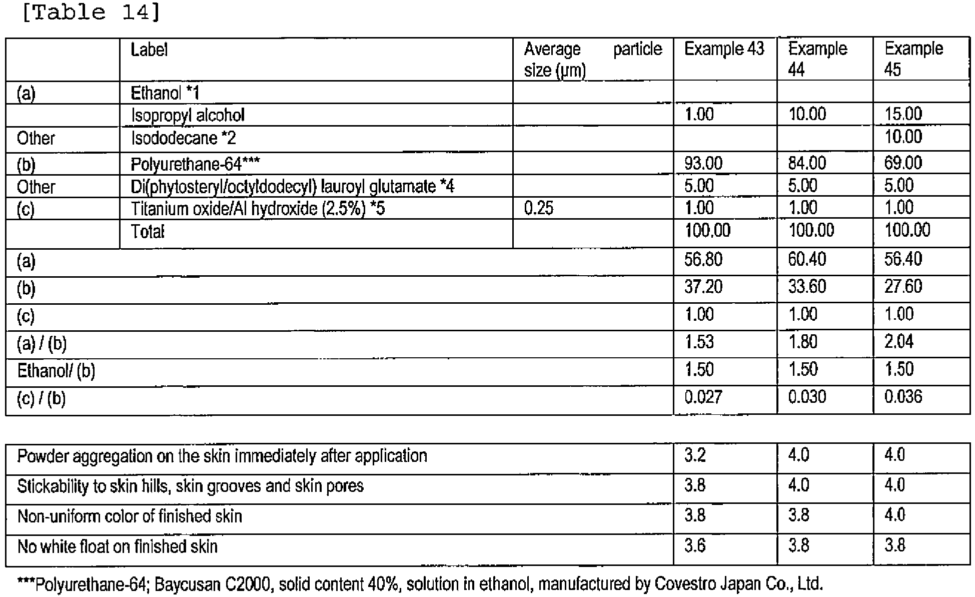

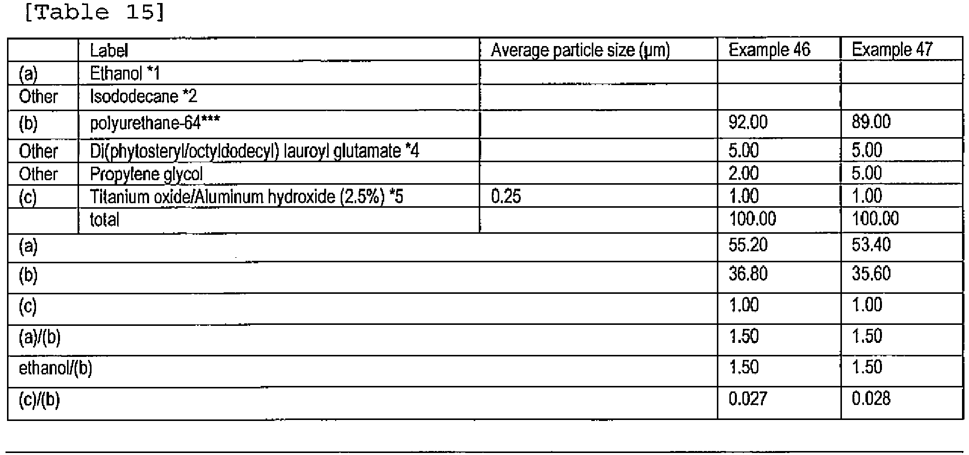

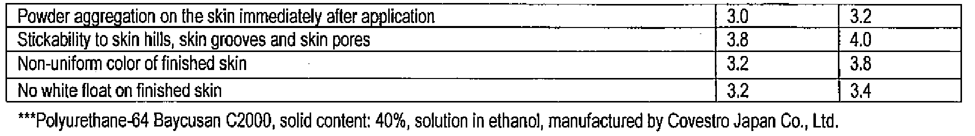

### [Description of Symbols]

- 10: Electrostatic spraying device
- 11: Low-voltage power supply
- 12: High-voltage power supply
- 13: Auxiliary electrical circuit
- 14: Microgear pump
- 15: Container
- 16: Nozzle
- 17: Pipe
- 18: Flexible pipe
- 19: Current limit resistor
- 20: Housing

## Claims

1. A method for producing a coating on skin, comprising a step of electrostatically spraying a composition comprising component (a), component (b) and component (c) directly onto the skin:
(a) one or more volatile substances selected from the group consisting of water, an alcohol and a ketone,
(b) a polymer having a film-forming ability, and
(c) a powder.

2. The method according to Claim 1, wherein a porous coating is formed on the skin by the electrostatically spraying onto the skin directly.

3. The method according to Claim 1 or 2, wherein the electrostatic spraying is performed by using an electrostatic spraying device having a container containing the composition, a nozzle for discharging the composition, a supply device for supplying the composition contained in the container to the nozzle, and a power supply for applying a voltage to the nozzle.

4. The method according to any one of Claims 1 to 3, wherein component (a) is one or more selected from the group consisting of ethanol, isopropyl alcohol, butyl alcohol, and water.

5. The method according to any one of Claims 1 to 4, wherein component (a) is a volatile substance comprising at least ethanol.

6. The method according to any one of Claims 1 to 5, wherein component (a) is a volatile substance comprising at least ethanol, and the content of ethanol in the volatile substance is 50 mass% or more and 100 mass%.

7. The method according to any one of Claims 1 to 6, wherein the content of component (a) is 30 mass% or more and 98 mass% or less in the composition.

8. The method according to any one of Claims 1 to 7, wherein component (b) is one or more selected from the group consisting of a fully saponified or partially saponified polyvinyl alcohol, a polyvinyl butyral resin, a polyurethane resin, an oxazoline-modified silicone, a water-soluble polyester, and Zein.

9. The method according to any one of Claims 1 to 8, wherein component (b) is one or more selected from the group consisting of a polyvinyl butyral resin and a polyurethane resin.

10. The method according to any one of Claims 1 to 9, wherein the content of component (b) is 2 mass% or more and 50 mass% or less in the spraying composition.

11. The method according to any one of Claims 1 to 10, wherein the content ratio of component (a) to component (b), ((a) / (b)), is 0.5 or more and 40 or less.

12. The method according to any one of Claims 5 to 11, wherein the content ratio of ethanol (a) to component (b), ((a)/(b)), is 0.5 or more and 40 or less.

13. The method according to any one of Claims 1 to 12, wherein component (c) is one or more selected from the group consisting of a coloring pigment, a constitutive pigment, a pearl pigment and an organic powder.

14. The method according to any one of Claims 1 to 13, wherein the content of component (c) is 0.001 mass% or more and 50 mass% or less in the composition.

15. The method according to any one of Claims 1 to 14, wherein the content ratio of component (c) to component (b), ((c)/(b)), is 0.001 or more and 20 or less.

16. The method according to any one of Claims 1 to 15, wherein the composition further comprises a polar oil in a liquid state at 20°C.

17. The method according to Claim 16, wherein the polar oil in a liquid state at 20°C is one or more selected from the group consisting of a hydrocarbon oil, an ester oil, an ether oil, a higher alcohol, and a silicone oil.

18. The method according to any one of Claims 1 to 17, wherein the composition comprises a glycol in a content of 10 mass% or less.

19. The method according to any one of Claims 1 to 18, wherein the composition is used as a cosmetic selected from the group consisting of a makeup cosmetic, a UV protection cosmetic, and a skincare cosmetic .

20. The method according to any one of Claims 1 to 19, wherein the content of component (a) in the composition is 30 mass% or more and 98 mass% or less, the content of component (b) in the spraying composition is 2 mass% or more and 50 mass% or less, and the content of component (c) in the spraying composition is 0.001 mass% or more and 50 mass% or less.

21. The method according to any one of Claims 1 to 19, wherein the content of component (a) is 55 mass% or more and 96 mass% or less in the composition, the content of component (b) is 4 mass% or more and 45 mass% or less in the spraying composition, and the content of component (c) is 0.01 mass% or more and 30 mass% or less in the spraying composition.

22. The method according to any one of Claims 1 to 19, wherein the content of component (a) is 60 mass% or more and 94 mass% or less in the composition, the content of component (b) in the spraying composition is 6 mass% or more and 40 mass% or less, and the content of component (c) in the spraying composition is 0.1 mass% or more and 15 mass% or less.

23. The method according to any one of Claims 1 to 22, wherein component (a) is a volatile substance comprising at least ethanol, and component (b) is one or more selected from the group consisting of a polyvinyl butyral resin and a polyurethane resin.

24. The method according to any one of Claims 1 to 23, wherein component (a) is a volatile substance comprising at least ethanol, and the composition further comprises up to 10 mass% of a glycol.

25. The method according to any one of Claims 1 to 24, wherein component (a) is a volatile substance comprising at least ethanol, the content of the ethanol in the volatile substance is 50 mass% or more and 100 mass%, and the composition comprises up to 10 mass% of a glycol.
